# EUROPEAN PATENT SPECIFICATION

(11) **EP 4 447 793 B1**
(45) Date of publication and mention of the grant of the patent: **20.05.2026**
(21) Application number: 22839164.5
(22) Date of filing: 13.12.2022
(51) Int. Cl.: A61B 5/0205, A61B 5/00

(54) **A SLEEP TRACKING SYSTEM**
SCHLAFVERFOLGUNGSSYSTEM
SYSTÈME DE SUIVI DU SOMMEIL

(30) Priority: 13.12.2021 GB 202117951
(43) Date of publication of application: 23.10.2024
(60) Divisional application: 26171521.3
(73) Proprietor: Willow Blossom Holdco Limited, London, Greater London EC2V 6DN (GB)
(72) Inventor: CARR, Andrew, London EC1N 8LE (GB); HAMMOND, Oxana, London EC1N 8LE (GB)
(74) Representative: Williams Powell
(86) International application number: PCT/EP2022/085687
(87) International publication number: WO 2023/110917

(56) References cited:
- WO-A1-2015/008285
- WO-A1-2017/139437
- WO-A1-2020/193842
- US-A1- 2011 267 196
- US-A1- 2019 236 925
- US-A1- 2021 190 351

## Description

### FIELD OF THE INVENTION

The present invention relates to a sleep tracking system.

### BACKGROUND

Less than 7 hours of sleep in 24 hours is associated with poorer general health and increased risk of disease.
- Long term sleep deprivation can be a factor in a plethora of health problems.
- There are also immediate effects on cognitive function.

By 3 years of age, the typical child has spent more time sleeping than in all wakeful activities combined.
- Infants younger than two months sleep on average 15 hours a day.
- Sleep is irregular.
- As the child ages their sleep becomes more regular and more consolidated.

Women are almost twice as likely as men to have poor quality of sleep.
- Women tend to sleep longer than men, but are also more likely to suffer from sleep disruptions.
- Menstrual cycle.
- Pregnancy.
- Childcare.
- Menopause.

There is a substantial amount of research on the great benefits of napping in populations with and without sleep deprivation.

There is a need to provide a system to solve these problems.

WO 2020/193842 A1 describes a platform with many sensors, sensor sheet, a transducer to produce acoustic and mechanical vibrations and an airflow blower. Sensors are also fixed to the mother and in the care room. A sleep/activity graph of the baby and sleep/emotion graph of the mother are measured and tracked with multimodal sensors and an artificial intelligence unit processes audio, motion and airflow actions given to the baby from the platform to help the baby to move from an improper to optimal sleep/activity level.

US 2021/190351 A1 describes a system for alerting a second subject (e.g. a parent) based on a wellbeing state of a first subject (e.g. a child) when the first subject is sleeping in the same room as the second subject. An arrangement of sensors is used for sensing data from the first subject and a processor is configured to determine the presence of the first subject from the sensing data.

US 2019/236925 A1 describes a system comprising an evaluation unit that allows to determine an occurrence of an event and, when the event occurs, to alert a plurality of persons and/or to inform them about the occurred event. The system comprises for each person a control unit and a sensor. The control unit can be connected to the evaluation unit for exchanging data.

WO 2015/008285A1 describes apparatus for use with a subject who shares a bed with a second person. A motion sensor detects motion of the subject and motion of the second person, and generates a motion signal in response thereto. A control unit analyzes the motion signal, in response thereto, identifies an effect of large body-movement of the second person on sleep of the subject, and in response thereto, generates a sleep-disturbance output.

US 2011/267196 A1 describes a system and method for providing sleep quality feedback that includes receiving alarm input on a base device from a user; the base device communicating an alarm setting based on the alarm input to an individual sleep device; the individual sleep device collecting sleep data based on activity input of a user; the individual sleep device communicating sleep data to the base device; the base device calculating sleep quality feedback from the sleep data; communicating sleep quality feedback to a user; and the individual sleep device activating an alarm, wherein activating the alarm includes generating tactile feedback to the user according to the alarm setting.

WO 2017/139437 A1 describes systems and methods for pumping milk from a breast, wherein the milk is expressed from the breast under suction and milk is expulsed from the pumping mechanism to a collection container under positive pressure.

### SUMMARY

There is provided a sleep tracking system as defined in the appended claims.

### BRIEF DESCRIPTION OF THE DRAWINGS

Specific embodiments are described below by way of example only and with reference to the accompanying drawings in which:
Figure 1 shows a sleep tracking system according to an embodiment of the invention.
Figure 2 shows an adult sleep tracker device according to an embodiment of the invention.
Figure 3 shows a baby monitor or baby sleep tracking device according to an embodiment of the invention.
Figure 4 shows the sleep analysis or sleep timing algorithm according to an embodiment of the invention.
Figure 5 shows a sleep tracking system according to an embodiment of the invention.
Figure 6 shows a sleep tracking system according to an embodiment of the invention.
Figure 7 shows a sleep tracking system according to an embodiment of the invention.
Figure 8 shows a sleep tracking system according to an embodiment of the invention.
Figure 9 shows an adult sleep tracker device according to an embodiment of the invention.
Figures 10A and 10B show an adult sleep tracker device according to an embodiment of the invention.
Figures 11A and 11B show an adult sleep tracker device according to an embodiment of the invention.
Figure 12 shows data processing steps involved in the sleep tracking system according to an embodiment of the invention.
Figure 13 shows a device, processor and memory according to any device described herein.

Aspects and features of the present invention are set out in the accompanying claims.

### DETAILED DESCRIPTION OF THE EMBODIMENTS

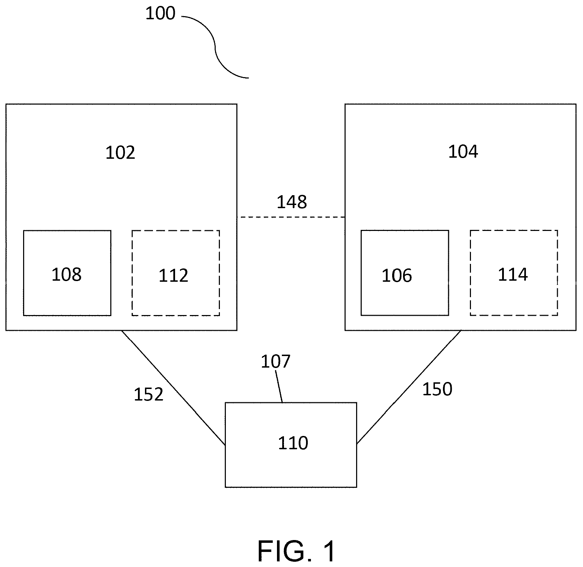

The present invention relates to a sleep tracking system. The optional features of the sleep tracking system will be described in the most detail with reference to Figure 1. The same features mentioned with respect to other Figures may operate in a similar way as described in relation to Figure 1.

### Key Feature A: Smart sleep tracking + sleep advice syste

A sleep tracking system configured to track and learn an adult's sleep patterns and to generate and display advice, based on those learnt sleep patterns, to that adult on when to schedule sleep, in order to increase the amount of sleep enjoyed by that adult.

Optional features:
Adult sleep tracker
- system includes an adult sleep tracker device that learns the adult's sleep patterns by tracking the time when the adult starts their sleep routine and/or when they start to sleep, and when they wake up, and generates a predictive model of that adult's sleep patterns and updates that predictive model on the basis of newly tracked sleep patterns
- adult sleep tracker device learns an adult's sleep patterns using a sleep sensing sub-system
- the sleep sensing sub-system is a contactless sleep tracking sub-system
- the sleep sensing sub-system is a contact-based sleep tracking sub-system, such as a sensor placed over a mattress, under a mattress, under or on a pillow
- sleep pattern includes one or more of: time asleep, time of going to bed, time to fall asleep, time actually asleep, snoring time, time awake in bed, time away from bed, wake-up time
- the contactless sleep tracking sub-system detects one or more of: movement of the adult using a radar, sonar or LIDAR system; breathing sounds using a microphone; algorithmic analysis of video
- the contactless sleep tracking sub-system detects one or more of breathing pattern, respiratory rate or heart rate
- adult sleep tracker device includes a multi-coloured light source and the colour at a given time indicates the scheduled recommendation for the adult at that time, such as sleeping or active.
- adult sleep tracker device includes a light source to aid sleep or relaxation or awakening, that can be physically adjusted to increase or decrease the amount of light visible, to make the light localised to one user, or to fill the whole room
- adult sleep tracker device includes a light source which can pulsate / expand and contract rhythmically to guide breathing and relaxation through a light 'metronome' effect
- adult sleep tracker device includes a speaker to generate sounds to aid sleep or relaxation or awakening, such as metronomic sounds and white noise.

Baby monitor or sleep tracker
- the system includes a baby monitor or sleep tracking device that detects if the baby is crying and then automatically plays soothing sounds
- the baby monitor or sleep tracking device detects if the baby is crying and then automatically turns on or increases the brightness of a light
- the baby monitor or sleep tracking device detects if the baby is settling and then automatically turns down the sounds and/or light gradually.
- the baby monitor or sleep tracking device automatically alerts the adult that the baby is crying only if the baby continues to cry for more than a pre-set or a learnt time, for example via phone or adult sleep device.
- baby monitor or sleep tracking device automatically alerts the adult if the baby continues to cry beyond the time that the baby monitoring device is normally able to, or has previously learnt that it is able to, automatically and autonomously settle a crying baby back to sleep
- the baby monitor or sleep tracking device includes button(s) that can be pressed to indicate the start and end of periods of sleep, and this information is shared with the app automatically
- the baby monitor or sleep tracking device automatically pauses sleep tracking when crying is detected, and resumes once the baby is settled
- the baby monitor or sleep tracking device learns the baby's sleep patterns by tracking the time when the baby starts to cry, and how long they take to settle themselves without adult comforting, and generates a predictive model of the baby's sleep patterns and/or self-settling patterns and updates that predictive model on the basis of newly tracked sleep patterns and/or self-settling patterns.
- the baby monitor or sleep tracking device detects or is informed when the baby is being fed or changed and records the time when that takes place.
- the baby monitor or sleep tracking device can play white noise or soothing sounds to help settle the baby to sleep in the first instance.
- the light on the baby monitor or sleep tracking device can be used as a low level night light, with brightness or colour tone changing when crying is detected.
- baby monitoring or sleep tracking through manual input by parent, or automated methods such as radar, Lidar, ballistocardiography, algorithmic video analysis.
- baby monitor or sleep tracking device learns to associate the cry of the baby with the baby's needs such as hunger, needs a diaper change, tiredness or distress
- baby monitor or sleep tracking device learns whether to automatically settle the crying baby back to sleep and/or to provide an alert to the adult regarding the baby's needs
- alert is provided to an adult via an app based on the baby's needs and the adult schedule such as 'pick up diaper en route'.

App
- the application enables the adult to input any of the following: demographic information, wellbeing and health habits, hormonal cycle, personalised routines, sleep goals, sleep and activity schedules.
- the system includes an adult sleep tracking device and an app, such as a smartphone app, that is connected to the adult sleep tracking device, and the app displays the sleep scheduling advice
- sleep scheduling advice includes specific times during the day or night during which sleep is or is not recommended, based on information known about the adult, for example circadian rhythm
- sleep scheduling advice includes specific times during the day or night during which activity is or is not recommended
- sleep scheduling advice includes specific times during the day or night during which drugs such as caffeine, alcohol or nicotine is or is not recommended
- sleep scheduling advice includes advice on how long to sleep for a time period such as a 24h time period
- the system includes baby monitor or sleep tracking device and an app, such as a smartphone app, that is connected to the baby monitor or sleep tracking device.
- the application displays the advice in a daily diary format showing times that sleep, such as naps and night-time sleep are recommended for the adult
- the application also displays the predicted sleep schedule of a baby
- the application, for the current day, shows when the baby awoke and when his next nap is scheduled
- the application, for the current day, shows when the baby awoke and when his next nap is scheduled
- the application allows for manual inputs of baby sleep data, for example when a baby has slept in a car seat away from their baby monitor or sleep tracking device
- the application, for the current day, shows various sleep insights, for example: when the adult woke in the morning, how long they slept for, how long it took for them to get to sleep, and when the adult should aim to sleep next
- the application displays the predicted sleep schedule of a baby in a daily diary format showing times that sleep is predicted, alongside the advice to the adult on when sleep for that adult is recommended
- the application displays the predicted sleep duration for the adult, based on expected sleep duration for the baby
- the application is provided with the birthdate or age of the baby and automatically generates sleep scheduling advice based on the standard developmental phases of the baby
- the application is provided with the birthdate or age of the baby and automatically generates general best-practise sleep advice based on the standard developmental phases of the baby

Advice
- advice is based on a sleep analysis or sleep timing algorithm that aims to increase the quantity of sleep enjoyed by that adult.
- the sleep analysis or sleep timing algorithm aims to increase the quantity of sleep enjoyed by that adult, and decrease the likelihood of sleep inertia by waking them from sleep when the device predicts that they are in light-sleeping phase of their natural sleep cycle.
- Sleep window advice is adjusted based on feedback or ratings provided by the user, so it learns what advice is helpful/successful for that user over time, for example 'how do you feel after your nap?'
- predictive advice relates to night-time sleep
- predictive advice relates to day-time nap or naps
- the predictive advice includes when to schedule periods of activity, based on understanding of how much sleep the individual has had
- predictive advice takes into account the adult's hormonal changes, as determined automatically by a hormone tracking or analysis system or data input by the adult
- predictive advice takes into account diary or activity inputs from, or associated with, the adult
- activity inputs are derived from a breast pump used by the adult
- activity inputs are derived from a wearable device, such as an activity tracker or smart watch, worn by the adult
- predictive advice takes into account when a baby wakes or cries, as determined by a baby monitoring device
- predictive advice takes into account how long it takes for a baby monitoring device to automatically and autonomously settle a crying baby back to sleep
- Algorithm which supports getting women to sleep faster, by learning their unique sleep patterns and adjusting programmes to reduce sleep onset time
- Algorithm which adjusts programmes or tips to women's sleep needs, e.g. suggesting longer sleep programmes if they're in a particular stage of their hormonal cycle which they find more difficult to fall asleep in, or tips such as suggesting to wear cooler clothes

### Description of Key Feature A: Smart sleep tracking + sleep advice system

In an embodiment of the present invention, a sleep tracking system 100 is provided. As depicted in Figure 1, the sleep tracking system 100 comprises one or more of: an adult sleep tracker device 102, baby sleep tracking device 104 and/or a control device 107 configured to run an application 110 (for example, a smartphone app). The control device 107 may be any device suitable to run an application.

The sleep tracking system 100 may be implemented over a network. The sleep tracking system 100 may be described as a sleep tracking ecosystem. The network comprises one or more devices: an adult sleep tracker device 102, a baby monitor or baby sleep tracking device 104 and/or a control device 107 which runs an application 110. Each of the one or more devices may be connected to each other via one or more communication links 148, 150, 152. In particular, communication link 148 is optional.

The communication links 148, 150, 152 may be configured to transfer information. The communication links 148, 150, 152 may be wired or wireless connections. For example, one or more of the communication links 148, 150, 152 may comprise a USB cable. One or more communication links 148, 150, 152 may comprise WiFi, bluetooth, or any other wireless connection.

The devices may be connected to each other (e.g., networked) in a Local Area Network (LAN), a Wide Area Network (WAN) for example, the Internet, or via any other network (e.g. Zigbee). The devices may each operate in the capacity of a server or a client machine in a client-server network environment, or as a peer machine in a peer-to-peer (or distributed) network environment.

The application 110 may run on a control device 107. The control device 107 comprises a processor. In other words, the sleep tracking system 100 may comprise a control device 107 configured to run application 110. The control device 107 may be a personal computer (PC), a tablet computer, a settop box (STB), a Personal Digital Assistant (PDA), a cellular telephone, a web appliance or a smartwatch. Of course any machine capable of displaying information and executing a set of instructions may be used.

The adult sleep tracker device 102 and baby monitor or baby sleep tracking device 104 may each comprise a processor, the processor configured to execute instructions.

The sleep tracking system 100 may be configured to track and learn an adult's sleep patterns and to generate and display advice, based on those learnt sleep patterns, to that adult on when to schedule sleep, in order to increase the amount of sleep enjoyed by that adult.

The sleep tracking system 100 may comprise a sleep sensing sub-system 108. The adult sleep tracker device 102 may comprise the sleep sensing sub-system 102. The sleep tracking system 100 is configured to track an adult's sleep patterns via the sleep sensing sub-system 108. The sleep sensing sub-system 108 is described in more detail below.

Advice may be generated by a sleep analysis or sleep timing algorithm. The advice may be displayed via the application 110. The sleep analysis or sleep timing algorithm and application 110 are described in more detail below.

The sleep tracking system 100 may be configured to track and learn an baby's sleep patterns and to generate and display advice, based on those learnt sleep patterns, to an adult on when to schedule sleep (adult and/or baby sleep), in order to increase the amount of sleep enjoyed by that adult and/or baby.

The adult sleep tracker device 102 may be used as a stand-alone device. That is, the baby sleep tracking device 104 is not required for the adult sleep tracker device 102 to provide some of its functions. This is advantageous because a parent may continue to use the adult sleep tracker device 102 even after baby sleep tracking is no longer required.

### Adult sleep tracker

The adult sleep tracker device 102 is configured to learn the adult's sleep patterns. The adult sleep tracker device 102 may be configured to learn the adult's sleep pattern using a sleep sensing subsystem. In some examples, sleep patterns may include one or more of: time asleep, time of going to bed, time to fall asleep, time actually asleep, time in sleep stages such as light sleep, REM and deep sleep, snoring time, periods of movement, time awake in bed, time away from bed, wake-up time.

In the present embodiment, the adult sleep tracker device 102 learns the adult's sleep patterns by tracking the time when the adult starts their sleep routine and/or when they start to sleep and/or when they enter different stages of sleep, and when they wake up. That is, the adult sleep tracker device 102 measures the time that the adult is asleep and the time of day that an adult typically falls asleep and wakes up.

The adult sleep tracker device 102 is configured to generate a predictive model of that adult's sleep patterns and to update the predictive model on the basis of newly tracked sleep patterns. The predictive model is based on the sleep analysis or sleep timing algorithm.

The adult sleep tracker device 102 is configured to learn an adult's sleep patterns using the sleep sensing sub-system 108. The sleep sensing sub-system 108 is configured to collect input data. Input data may comprise adult sleep pattern information. Input data may define the adult's sleep patterns. Adult sleep pattern information (input data) may comprise one or more of: time asleep, time of going to bed, time to fall asleep, time actually asleep, time in sleep stages such as light sleep, REM and deep sleep, snoring time, periods of movement, time awake in bed, time away from bed, wake-up time .

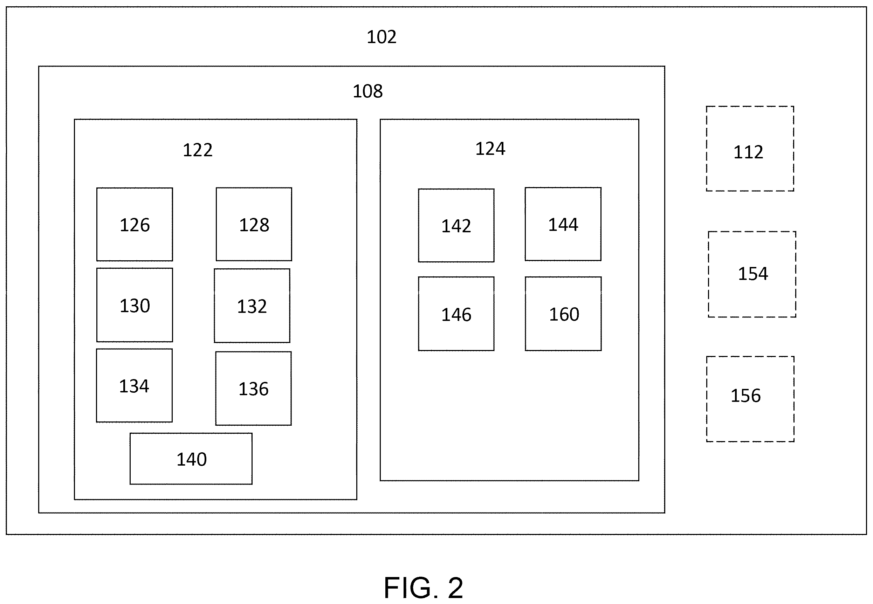

Figure 2 depicts the adult sleep tracker device 102 according to one embodiment. The sleep sensing sub-system 108 may include one or more of: a contactless sleep tracking sub-system 122 and a contact sleep tracking sub-system 124.

The contactless sleep tracking sub-system 122 may be placed away from the adult, for example on a bedside table or affixed elsewhere in the adult's bedroom. The contactless sleep tracking sub-system 122 may detect one or more of: movement of the adult using a radar, sonar or LIDAR system; breathing sounds using a microphone; algorithmic analysis of video. Accordingly, the contactless sleep tracking sub-system 122 may comprise one or more of: a radar system 126, a sonar system 128, a LIDAR system 130, a microphone 132 and a camera 136. Additionally or alternatively, the contactless sleep tracking sub-system 122 may comprise one or more of: a heart rate sensor 134 and a ballistocardiogram-based sensor 140.

In a first embodiment, the contactless sleep tracking sub-system 122 comprises a radar system 126. The radar system 126 comprises a transmitter for transmitting radio signals and a receiver for receiving radio signals. The signals provide information on the position and distance to the object (i.e. the adult in their bed) by calculating the time of flight. The contactless sleep tracking sub-system 122 is therefore able to detect if an adult is located in their bed or not. Continued measurement also allows motion information to be obtained. For example, a rapidly changing position of the adult may indicate that the adult is awake while little to no movement of the adult may indicate that the adult is asleep. Therefore the radar system 126 may provide adult sleep pattern information.

In a second embodiment, the contactless sleep tracking sub-system 122 comprises a sonar system 128. The sonar system 128 comprises a transmitter for transmitting sound signals and a receiver for receiving sound signals. The signals provide information on the position and distance to the object (i.e. the adult in their bed) by calculating the time of flight. Continued measurement also allows motion information to be obtained. Therefore the sonar system 128 may provide adult sleep pattern information.

In a third embodiment, the contactless sleep tracking sub-system 122 comprises a LIDAR system 130. The LIDAR system 130 comprises a transmitter for transmitting laser signals and a receiver for receiving laser signals. The signals provide information on the position and distance to the object (i.e. the adult in their bed) by calculating the time of flight. Continued measurement also allows motion information to be obtained. Therefore the LIDAR system 130 may provide adult sleep pattern information. Any other suitable time of flight sensor or similarly operating sensor may also be used.

The contactless sleep tracking sub-system may detect one or more of: breathing pattern, respiratory rate or heart rate.

In a fourth embodiment, the contactless sleep tracking sub-system 122 comprises a microphone 132. Of course, any suitable audible sensor may be used. The microphone may be configured to detect one or more of: breathing pattern and respiratory rate. The breathing pattern may include one or more of: regularity of breaths, frequency of breaths, depth of breaths. Respiratory rate is the frequency of breathing. The breathing pattern and respiratory rate provide adult sleep pattern information. For example, a slow and uniform breathing pattern indicates that the adult is asleep whereas shallow and faster breathing may indicate that the adult is awake.

In a fifth embodiment, the contactless sleep tracking sub-system 122 comprises a heart rate sensor 134. The heart rate sensor 134 may comprise a radar sensor configured to detect an adult heartbeat. The heart rate sensor 134 may provide adult sleep pattern information. For example, a slower heartrate indicates that the adult is asleep whereas faster heartrate may indicate that the adult is awake.

In a sixth embodiment, the contactless sleep tracking sub-system 122 comprises a camera 136. The contactless sleep tracking sub-system may be configured to detect movement of the adult via an algorithmic analysis of video. The camera may be configured to record video wherein algorithmic analysis of the video provides adult sleep pattern information.

In a seventh embodiment, the contactless sleep tracking sub-system 122 comprises a ballistocardiogram-based sensor 140. The ballistocardiogram-based sensor 140 may use ballistocardiography to provide adult sleep pattern information. The ballistocardiogram-based sensor 140 may be configured to detect movement of the adult. The ballistocardiogram-based sensor 140 may be a force sensor placed under the mattress of the adult's bed.

The sleep sensing sub-system 108 may comprise the contact-based sleep tracking sub-system 124. The contact-based sleep tracking sub-system 124 may comprise one or more sensors located in one or more positions, the positions including: the topside of a mattress, the underside of a mattress, the topside on a pillow, the underside of a pillow and inside a pillowcase. The contact-based sleep tracking sub-system 108 may be one or more of: one or more movement sensors 142, one or more pressure sensors 144, one or more temperature sensors 146 and one or more heart rate sensors 160

In a first embodiment, the contact-based sleep tracking sub-system 108 comprises one or more movement sensors 142. The one or more movement sensors may be configured to provide movement information about the adult and therefore to provide adult sleep pattern information.

In a second embodiment, the contact-based sleep tracking sub-system 108 comprises one or more pressure sensors 144. The one or more pressure sensors may be configured to provide movement information about the adult and therefore to provide adult sleep pattern information.

In a third embodiment, the contact-based sleep tracking sub-system 108 comprises one or more temperature sensors 146. The one or more temperature sensors may be configured to detect a change in the adult's body temperature. A drop in body temperature is associated with an adult being asleep and therefore the one or more temperature sensors provide adult sleep pattern information.

In a fourth embodiment, the contact-based sleep tracking sub-system 108 comprises one or more heart rate sensors 160. The heart rate sensor 160 may be configured to detect a change in the adult's heart rate. A change in the adult's heart rate may provide adult sleep pattern information.

The contactless or contact-based sleep tracking sub-system 122, 124, may be configured to be calibrated. Calibration provides improved precision in adult sleep pattern information. The contactless or contact-based sleep tracking sub-system 122, 124 may be configured to operate in a calibration mode. During calibration (in the calibration mode) the adult is awake and lies in their normal sleeping position. The sleep sensing sub-system 108 may be configured to calibrate where the adult lies in their normal sleeping position. Any sensor described herein may be calibrated in this way to gain reference data. For example, the radar system 126 may be calibrated by taking data when the adult is lying in their bed and when the adult is out of their bed.

The adult sleep tracker device 102 may include button(s) 156 that can be pressed to indicate the start and end of periods of sleep.

Although Figure 2 is depicted and described with reference to the adult sleep tracker device 102, the baby monitor or baby sleep tracking device 104 may comprise a similar structure.

### Light source

The adult sleep tracker device 102 optionally includes a multi-coloured light source 112 and the colour at a given time indicates the scheduled recommendation for the adult at that time, such as sleeping or active. In other words, the adult sleep tracker device 102 may comprise light source 112.

The sleep tracking system 100 may be configured to execute the sleep analysis or sleep timing algorithm. The sleep analysis or sleep timing algorithm may generate predictive advice. The predictive advice may comprise the scheduled recommendation. The predictive advice may comprise one or more of scheduled recommendations. The scheduled recommendation may include a recommendation for: the adult to sleep, the adult to be awake, the adult to start to wind down for sleep, the adult to do physical activity, the adult to feed their baby, the adult to put their baby to bed and the adult to comfort their baby.

The light source may be configured such that the colour is associated to a scheduled recommendation. In other words, the light source may be configured to light a colour based on a scheduled recommendation. For example, if the scheduled recommendation is for the adult to sleep, the light source is configured to be a first colour and if the scheduled recommendation is for the adult to be awake then the light source 112 is configured to be a second colour.

The adult sleep tracker device 102 may include the light source 112 to aid sleep or relaxation or awakening, that can be physically adjusted to increase or decrease the amount of light visible, to make the light localised to one user, or to fill the whole room.

In a first embodiment, the light source 112 may be configured to gradually increase its brightness when the adult is scheduled to wake up. The light source 112 gradually wakes up the adult by mimicking the rising of the Sun. This method of waking the adult is advantageous because, by mimicking the rising of the Sun, the adult wakes up feeling refreshed. Mimicking the rising of the Sun may be based on the adult's location and/or the date. In different countries and at different times of year, the Sun rises at different times and the rising of the Sun may take a longer or shorter period of time. The light source 112 may be configured to maximise a match between the mimicking of the rising of the Sun with the actual rising of the Sun, based on the adult's location and/or the date.

Additionally or alternatively, in a second embodiment, the light source 112 may be configured to gradually decrease its brightness when the adult is scheduled to go to sleep. This mimics the Sun setting and gradually prepares the adult for their sleep. Again, the light source 112 may be configured to maximise a match between the mimicking of the setting of the Sun with the actual setting of the Sun, based on the adult's location and/or the date.

In a third embodiment, the light source 112 may be configured to be localised. In this embodiment, the light source 112 may be movable. For example, the light source may be able to be twisted, rotated, pulled or pushed in order to manipulate the direction of the light by the user. In this embodiment the light source may extend out of the surface of the adult sleep tracker device, attached to the body of the adult sleep tracker device via an attachment portion. In this embodiment, the light source 112 user allows that user to relax, for example by allowing the user to use the light source to read a book.

In a fourth embodiment, the adult sleep tracker device 102 may include the light source 112 which can pulsate / expand and contract rhythmically to guide breathing and relaxation through a light 'metronome' effect. In other words, the light source 112 is configured to execute breathing exercises for the adult by changing brightness. The light source 112 may be configured to "expand" by increasing the light brightness. The light source 112 may be configured to "contract" by decreasing the light source brightness. The light source 112 may be configured to execute a light metronome effect. The light metronome effect is a regular timed change in the brightness of the light source 112 or the light colour.

Regular (i.e. pulsating / rhythmic) changes in light brightness and/or light colour by the light source 112 indicate to the adult to inhale for a defined time period and exhale for a defined time period. The light source 112 may oscillate or vary in intensity with a period that, if the time period is matched by a person's breathing rate, tends to relax that person. For example, the light source 112 may start turned off and then gradually increase brightness (expand) to indicate that the adult should inhale in this time period, the light may then gradually decrease brightness (contract) to indicate that the adult should exhale in this time period. Alternatively or additionally, the light may be set to a first colour to indicate that the adult should inhale in this time period and the light may change to a second colour to indicate that the adult should exhale in this time period. The inhale and exhale periods may be predefined time periods which repeat in a regular manner. For example, the inhale period may be 5 seconds and the exhale period 10 seconds. In another example, the inhale period may be 6 seconds and the exhale period 8 seconds.

Additionally or alternatively, in a fifth embodiment, the adult sleep tracker device 102 may comprise an audio source. The audio source 154 may be in a similar manner to the light source 112. The audio source may be configured to oscillate or vary in sound intensity with a period that, if matched by a person's breathing rate, tends to relax that person. The audio source 154 may be configured to execute breathing exercises for the adult by varying outputted sounds.

The adult sleep tracker device 102 may dim or turn the light source 112 and/or audio source 154 gradually off when it detects that the person has fallen asleep, or exhibits signs, such as altered breathing rate, that indicates that the person will shortly fall asleep.

The adult sleep tracker device 102 may include a speaker to generate sounds to aid sleep or relaxation or awakening, such as metronomic sounds and white noise. The speaker may generate various soundscapes (e.g. ocean waves) and/or narrated stories. The speaker may be the same component as the audio source 154 or be another component. Any sound to aid in relaxation or sleep or awakening may be used. The sound may be selected manually by the user. Sounds for relaxing and/or sleep may include sounds such as metronomic sounds, white noise, weather sounds or animal sounds. While sounds for awakening include sounds such as those used for alarms, e.g. the sounds of bells or a buzzer.

### Baby monitor or sleep tracker

The sleep tracking system 100 may include a baby monitor or sleep tracking device 104. A "baby monitor" may act in a similar manner to a "baby sleep tracking device". A baby monitor may be described as a baby sleep tracking device, the terms are interchangeable herein.

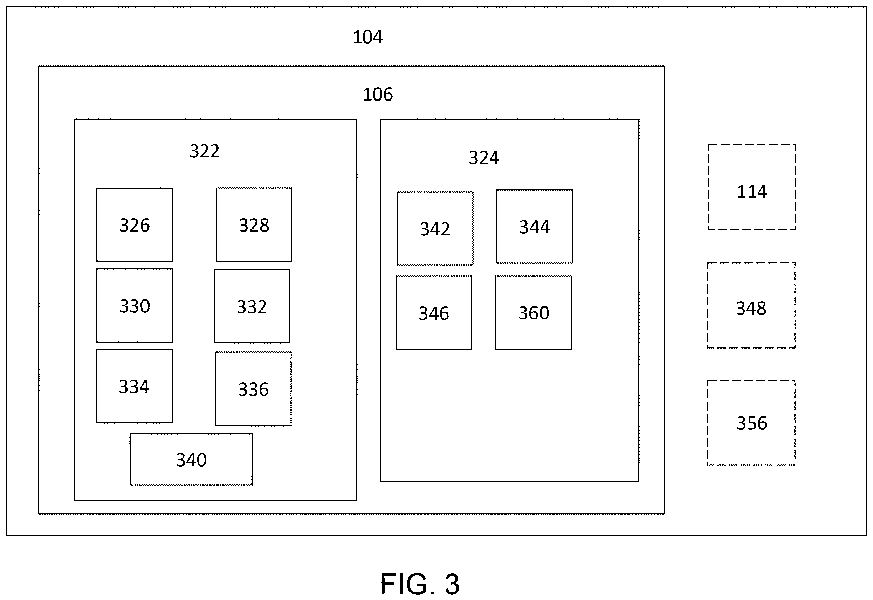

Figure 3 depicts the baby monitor or sleep tracking device 104. The baby monitor may comprise a baby sleep tracking sub-system 106. The baby sleep tracking sub-system 106 may detect the baby's sleep patterns. Optionally, the baby monitor or sleep tracking device 104 comprises a light source 114.

The baby monitor or baby sleep tracking device 104 may comprise a similar structure to the adult sleep tracker device described in relation to Figure 2. The sleep sensing sub-system 106 may be one or more of: a contactless sleep tracking sub-system 322 and a contact sleep tracking sub-system 324.

The contactless sleep tracking sub-system 322 may be placed away from the baby, for example on a bedside table or affixed elsewhere in the baby's bedroom. The contactless sleep tracking sub-system 322 may detect one or more of: movement of the baby using a radar, sonar or LIDAR system; breathing sounds using a microphone; algorithmic analysis of video. Accordingly, the contactless sleep tracking sub-system 322 may comprise one or more of: a radar system 326, a sonar system 328, a LIDAR system 330, a microphone 332 and a camera 336. Additionally or alternatively, the contactless sleep tracking sub-system 322 may comprise one or more of: a heart rate sensor 334 and a ballistocardiogram-based sensor 340.

In a first embodiment, the contactless sleep tracking sub-system 322 comprises a radar system 326. The radar system 326 comprises a transmitter for transmitting radio signals and a receiver for receiving radio signals. The signals provide information on the position and distance to the object (i.e. the baby in their bed) by calculating the time of flight. The contactless sleep tracking sub-system 322 is therefore able to detect if an baby is located in their bed or not. Continued measurement also allows motion information to be obtained. For example, a rapidly changing position of the baby may indicate that the baby is awake while little to no movement of the baby may indicate that the baby is asleep. Therefore the radar system 326 may provide baby sleep pattern information.

In a second embodiment, the contactless sleep tracking sub-system 322 comprises a sonar system 328. The sonar system 328 comprises a transmitter for transmitting sound signals and a receiver for receiving sound signals. The signals provide information on the position and distance to the object (i.e. the baby in their bed) by calculating the time of flight. Continued measurement also allows motion information to be obtained. Therefore the sonar system 328 may provide baby sleep pattern information.

In a third embodiment, the contactless sleep tracking sub-system 322 comprises a LIDAR system 330. The LIDAR system 330 comprises a transmitter for transmitting laser signals and a receiver for receiving laser signals. The signals provide information on the position and distance to the object (i.e. the baby in their bed) by calculating the time of flight. Continued measurement also allows motion information to be obtained. Therefore the LIDAR system 330 may provide baby sleep pattern information. Any other suitable time of flight sensor or similarly operating sensor may also be used.

The contactless sleep tracking sub-system may detect one or more of: breathing pattern, respiratory rate or heart rate.

In a fourth embodiment, the contactless sleep tracking sub-system 322 comprises a microphone 332. Of course, any suitable audible sensor may be used. The microphone may be configured to detect one or more of: breathing pattern and respiratory rate. The breathing pattern may include one or more of: regularity of breaths, frequency of breaths, depth of breaths. Respiratory rate is the frequency of breathing. The breathing pattern and respiratory rate provide baby sleep pattern information. For example, a slow and uniform breathing pattern indicates that the baby is asleep whereas shallow and faster breathing may indicate that the baby is awake.

In a fifth embodiment, the contactless sleep tracking sub-system 322 comprises a heart rate sensor 334. The heart rate sensor 334 may comprise a radar sensor configured to detect an baby heartbeat. The heart rate sensor 334 may provide baby sleep pattern information. For example, a slower heartrate indicates that the baby is asleep whereas faster heartrate may indicate that the baby is awake.

In a sixth embodiment, the contactless sleep tracking sub-system 322 comprises a camera 336. The contactless sleep tracking sub-system may be configured to detect movement of the baby via an algorithmic analysis of video. The camera may be configured to record video wherein algorithmic analysis of the video provides baby sleep pattern information.

In a seventh embodiment, the contactless sleep tracking sub-system 322 comprises a ballistocardiogram-based sensor 340. The ballistocardiogram-based sensor 340 may use ballistocardiography to provide baby sleep pattern information. The ballistocardiogram-based sensor 340 may be configured to detect movement of the baby. The ballistocardiogram-based sensor 340 may be a force sensor placed under the mattress of the baby's bed.

The sleep sensing sub-system 308 may comprise the contact-based sleep tracking sub-system 324. The contact-based sleep tracking sub-system 324 may comprise one or more sensors located in one or more positions, the positions including: the topside of a mattress, the underside of a mattress, the topside on a pillow, the underside of a pillow and inside a pillowcase. The contact-based sleep tracking sub-system 308 may be one or more of: one or more movement sensors, one or more pressure sensors, one or more temperature sensors and one or more heart rate sensors.

In a first embodiment, the contact-based sleep tracking sub-system 308 comprises one or more movement sensors 342. The one or more movement sensors may be configured to provide movement information about the baby and therefore to provide baby sleep pattern information.

In a second embodiment, the contact-based sleep tracking sub-system 308 comprises one or more pressure sensors 344. The one or more pressure sensors may be configured to provide movement information about the baby and therefore to provide baby sleep pattern information.

In a third embodiment, the contact-based sleep tracking sub-system 308 comprises one or more temperature sensors 346. The one or more temperature sensors may be configured to detect a change in the baby's body temperature. A drop in body temperature is associated with an baby being asleep and therefore the one or more temperature sensors provide baby sleep pattern information.

In a fourth embodiment, the contact-based sleep tracking sub-system 308 comprises one or more heart rate sensors 360. The heart rate sensor 360 may be configured to detected a change in the baby's heart rate. A change in the baby's heart rate may provide baby sleep pattern information.

The contactless or contact-based sleep tracking sub-system 322, 324, may be configured to be calibrated. Calibration provides improved precision in baby sleep pattern information. The contactless or contact-based sleep tracking sub-system 322, 324 may be configured to operate in a calibration mode. During calibration (in the calibration mode) the baby is awake and lies in their normal sleeping position. The sleep sensing sub-system 308 may be configured to calibrate where the baby lies in their normal sleeping position. Any sensor described herein may be calibrated in this way to gain reference data. For example, the radar system 326 may be calibrated by taking data when the baby is lying in their bed and when the baby is out of their bed.

The baby sleep tracking device 104 may include an automatic baby settling feature. The baby sleep tracking device 104 may be configured to perform the following sequential steps: detect that a baby is awake or crying, initiate a settling routine to settle the baby back to sleep, detect that the baby is no longer awake or crying, and stop the settling routine.

The baby monitor or sleep tracking device 104 may detect if the baby is crying and in response play soothing sounds from a speaker or audio source 348. The baby monitor or sleep tracking device 104 may be configured to detect if the baby is crying. The microphone 332 may be configured to detect if the baby is crying. The audio source 348 may be configured to automatically play soothing sounds. Soothing sounds may be any suitable sound (for example, white noise, rain, music, or pre-recorded sounds from a user).

The baby monitor or sleep tracking device 104 may detect if the baby is crying and in response, turn on or gradually increase the brightness of a light. The microphone 332 may be configured to detect if the baby is crying. The light source 114 may be configured to automatically turns on or increases the brightness of the light.

Soothing sounds and increased brightness of the light source 114 both advantageously comfort and settle the baby, therefore increasing the chance that the baby will stop crying, self-soothe and return to sleep. This may reduce the number of times that a parent will need to be disturbed from their sleep to intervene.

The baby monitor or sleep tracking device 104 may detect if the baby is settling and then automatically turn down the sounds and/or light gradually. In some examples, the sounds and/or light source turn down gradually until the sounds and/or lights are turned off. In other examples, the sounds and/or light source turn gradually down until they reach a low level which is maintained for a time period. The microphone 332 may be configured to detect if the baby is settling by detecting when the baby stops crying. This feature helps the baby return to sleeping once they have settled from crying.

The baby monitor or sleep tracking device 104 may also play white noise or soothing sounds to help settle the baby to sleep in the first instance. That is, the audio source 348 may be configured to play white noise or soothing sounds to help settle the baby to sleep. Getting the baby to sleep in the first instance refers to when they are laid down to rest in their bed by the adult.

The baby monitor or sleep tracking device 104 may automatically alert the adult that the baby is crying only if the baby continues to cry for more than a pre-set or a learnt time, for example via phone or adult sleep device. The baby monitor or sleep tracking device 104 may be configured to alert the adult by communicating an alert to the app 110 and/or the adult sleep tracker device 102. The alert to the adult may comprise a push notification via the app 110. The alert to the adult may comprise a noise outputted by the adult sleep tracker device 102 (e.g. generated by audio source 154).

The baby sleep tracking device may be configured to: detect that a baby is awake or crying; initiate a settling routine to settle the baby back to sleep; detect that the baby is still awake or crying after a pre-defined time threshold; trigger an alert at one or both of the adult sleep tracker device or the control device.

The pre-set time may be a pre-defined time period defined by the adult. The pre-set time may be a pre-defined period defined by default settings. For example, the pre-set time might be set to 2, 5, 10 or 15 minutes.

The learnt time may be generated based on previous baby crying time data (i.e. historical data). The sleep tracking system 100 may record the period of time the baby cried before the baby was successfully settled back to sleep and the period of time the baby cried before the baby was unsuccessfully settled back to sleep (where the adult intervened).

In some examples, the baby may be settled back to sleep automatically and autonomously by the sleep tracking system. In these examples, the sleep tracking system 100 may record the period of time the baby cried before the baby was successfully settled back to sleep by the sleep tracking system 100. In alternative examples, the baby may not be settled back to sleep either automatically and autonomously by the sleep tracking system, but they may settle themselves without adult comforting. In these examples, the sleep tracking system 100 may record the period of time the baby cried before the baby was un/successfully self-settled back to sleep.

In a first embodiment, the learnt time may be the maximum period of time the baby has cried before the baby was successfully settled back to sleep. In a second embodiment, the learnt time may be the average period of time the baby has cried before the baby was successfully settled back to sleep.

In some embodiments, the learnt time is based on data recorded throughout the entire lifetime of the sleep tracking system 100. Alternatively, the learnt time is based on data recorded over a defined time period of history of the device. For example, over the previous month of data or over the previous 2 months of data.

By way of example, the previous baby crying time data might indicate the baby has only successfully been settled back to sleep after a maximum of 5 minutes over the entire lifetime of the device. In this example, the learnt time is 5 minutes. Of course, the learnt time can change over time, this is advantageous because as the baby gets older and develops, its sleeping habits may change.

Reference will now be made to the example where the baby may be settled back to sleep either automatically and autonomously by the sleep tracking system 100. The baby monitor or sleep tracking device 104 may automatically alert the adult if the baby continues to cry beyond the time that the baby monitoring device is normally able to, or has previously learnt that it is able to, automatically and autonomously settle a crying baby back to sleep. The time that the baby monitoring device is normally able to, or has previously learnt that it is able to, automatically and autonomously settle a crying baby back to sleep is the "learnt time".

Reference will now be made to the example where the baby may settle themselves without adult comforting. The baby monitor or sleep tracking device 104 may learn the baby's sleep patterns by tracking the time when the baby starts to cry, and how long they take to settle themselves without adult comforting, and generates a predictive model of the baby's sleep patterns and/or self-settling patterns and updates that predictive model on the basis of newly tracked sleep patterns and/or self-settling patterns.

The baby monitor or sleep tracking device 104 may include button(s) 356 that can be pressed to indicate the start and end of periods of sleep.

The baby monitor or sleep tracking device 104 may automatically pause sleep tracking when crying is detected, and resumes once the baby is settled. That is, when the baby is detected as crying the baby is recorded as being awake and not asleep.

The baby monitor or sleep tracking device 104 may detect or is informed when the baby is being fed or changed and records the time when that takes place. The baby monitor or sleep tracking device 104 may detect when the baby is being fed or changed by detecting when the baby has moved out of their bed using the baby sleep tracking sub-system 106. The baby sleep tracking sub-system 106 may be configured to detect when the baby has moved out of their bed using one or more of the: radar system 326, sonar system 328, LIDAR system 330, microphone 332, heart rate sensor 334, camera 336 a ballistocardiogram-based sensor 340. In the embodiment where the microphone 332 is configured to detect when the baby is being fed or changed, detection of adult voice frequencies may indicate that the baby is being fed or changed. The system 100 may be informed the baby is being fed or changed by the adult pressing a button 356 or pressing a button on the app 110.

The light on the baby monitor or sleep tracking device may be used as a low level night light, with brightness or colour tone changing when crying is detected. That is, the light source 114 may be configured as a night light. Additionally or alternatively, the light source 114 may be configured to change colour when crying of the baby is detected. The light source 114 may be configured to a low level of brightness to encourage the baby to sleep. If the light source 114 is set to a high level of brightness, this may keep the baby awake. The light source may be configured such that the brightness of the light and/or colour tone changes when crying of the baby is detected. An increase in brightness or a change in colour helps to settle the baby by comforting the baby. Crying may be detected by the microphone 332.

The baby monitoring or sleep tracking may be through manual input by the parent, or by automated methods such as radar, Lidar, ballistocardiography, algorithmic video analysis. The baby monitor or baby sleep tracking device 104 may be configured to receive baby sleep pattern information via button(s) 356. For example, the adult may manually input when the baby has fallen asleep. Manual input (e.g. via button(s)) can act to calibrate the automated methods.

The baby monitor or sleep tracking device 104 may learn to associate the cry of the baby with the baby's needs such as hunger, needing a diaper change, tiredness or distress. This may be achieved during a learning process. In some examples, the system 100 is configured to operate in a "learning mode" to learn to associate the cry of the baby with the baby's needs. While configured to operate in a "learning mode", the system 100 is configured to detect when the baby is crying (i.e. via the microphone 332) and the adult is alerted. The adult then inputs into the system 100 (e.g. via buttons 356 or via the app 110) the baby's one or more needs. The microphone 332 is configured to detect characteristics of the baby's cry (frequency, volume and/or duration). In the learning mode, the sleep tracking system 100 is configured to pair the characteristics of the baby's cry data and the baby's one or more needs when both are recorded in the same crying session (i.e. at the same time period). The paired data may be stored as a look-up table. The system 100 is configured to pre-empt the baby's needs by detecting characteristics of the baby's cry and using the look-up table to output the baby's need/s. The baby's needs are what the baby needs to stop crying. The baby's needs may comprise one or more of: a feed, a diaper change and comfort. An alert comprising the determined baby's needs may be sent to the adult via the app 110 or via the adult sleep tracker device.

The baby monitor or sleep tracking device 104 may learn whether to automatically settle the crying baby back to sleep and/or to provide an alert to the adult regarding the baby's needs. That is, based on the baby's need inferred by the system 100, the system 100 is configured to either: settle the baby back to sleep (via the baby sleep tracking device 104) or to alert the adult via the adult sleep tracker device 102 or the app 110.

By way of example, if the detected baby's cry is associated with the baby's need for feeding, then the adult may be alerted quickly. This is because it is unlikely for the baby to fall back asleep whilst it is hungry. In another example, if the detected baby's cry is associated with tiredness or distress then then the system is likely to be successful to settle the baby (e.g. using the light or soothing sounds) and get the baby back to sleep. This provides improved efficiency in the system 100, the system 100 avoids disturbing the parent's sleep unnecessarily.

Additionally, an alert may be provided to an adult via the app 110 based on the baby's needs and the adult schedule such as 'pick up diaper en route'. If the baby's need for a diaper change is detected based on the baby's cry, then this alert may be sent to the adult.

### Data processing

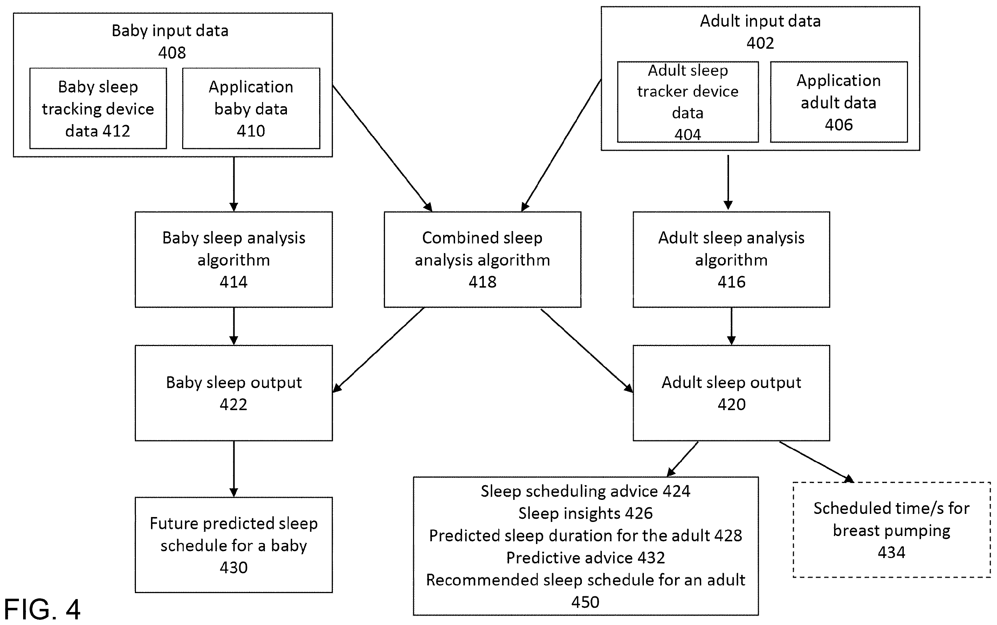

Reference will now be made to Figure 4. Figure 4 depicts one embodiment of the data processing steps involved in the sleep tracking system of the present invention. The sleep tracking system may be the system of any other Figure. Figure 4 depicts the generation of a coordinated sleep schedule.

Figure 4 depicts the sleep analysis or sleep timing algorithm. The sleep analysis or sleep timing algorithm may comprise one or more of: a baby sleep analysis algorithm 414, an adult sleep analysis algorithm 416 and a combined sleep tracking algorithm 418.

Baby input data 408 may comprise baby monitor or baby sleep tracking device data 412 and/or application baby data 410. Adult input data 402 may comprise adult sleep tracker device data 404 and/or application adult data 406. Baby input data 408 and/or adult input data 402 may pass to the combined sleep analysis algorithm 418. Baby input data 408 may pass to the baby sleep analysis algorithm 414. Adult input data 402 may pass to the adult sleep analysis algorithm 416. The combined sleep analysis algorithm 418 may generate a baby sleep output 422 and/or an adult sleep output 420. The baby sleep analysis algorithm 414 may generate baby sleep output 422. The adult sleep analysis algorithm 416 may generate adult sleep output 420. Baby sleep output 422 may comprise the predicted sleep schedule of a baby 430. Adult sleep output 420 may comprise one or more of: sleep scheduling advice 424, sleep insights 426, predicted sleep duration for the adult 428, predictive advice 432 and scheduled time/s for breast pumping 434.

The combined sleep tracking algorithm 418 may be used to generate a coordinated sleep schedule. Generating a coordinated sleep schedule may comprise: receiving adult input data 402; receiving baby input data 408, processing the adult input data and baby input data using key performance indicators; and generating an adult sleep output 420 and a baby sleep output 422.

The adult sleep analysis algorithm 416 receives adult input data 402. Adult input data 402 may comprise adult sleep tracker device data 404 and/or application data 406. Adult sleep tracker device data 404 is data captured by the adult sleep tracker device. Adult sleep tracker device data 404 may comprise one or more of: movement of the adult in their bed, breathing sounds, heartbeat, time waking up, time falling asleep, snoring time, total time asleep. Application adult data 406 is data captured by the application. For example, data inputted into the application by the adult.

The baby sleep analysis algorithm 414 receives baby input data 408. Baby input data 408 may comprise baby monitor or baby sleep tracking device data 412 and/or application data 406. Baby monitor or baby sleep tracking device data 412 is data captured by the baby monitor or baby sleep tracking device. Application baby data 410 is data captured by the application. For example, data inputted into the application by the adult.

The adult sleep analysis algorithm 416 processes adult input data 402 to generate adult sleep output 420. The baby sleep analysis algorithm 414 processes baby input data 408 to generate baby sleep output 422.

The baby sleep analysis algorithm 414, combined sleep analysis algorithm 418, adult sleep analysis algorithm 416 may process the input data by associating the data with key performance indicators. The key performance indicators may be one or more of: time of going to bed, time spent asleep, time to fall asleep, time actually asleep, time in sleep stages such as light sleep, REM and deep sleep, number of times the adult was woken up, time spent awake during the night, periods of movement, quality of sleep, wake up time. Over time, the algorithm(s) 418, 416, 414 use the key performance indicators to improve the resulting output.

In some embodiments, a combined sleep analysis algorithm 418 receives baby input data 408 and adult input data 402 to generated baby sleep output 422 and/or adult sleep output 420. The combined sleep analysis algorithm 418 allows synergy between baby sleep output 422 and/or adult sleep output 420.

The adult sleep output 420 may comprise one or more of: recommended sleep schedule for an adult 450, sleep scheduling advice 424, sleep insights 426, predicted sleep duration for the adult 428 and predictive advice 432. The recommended sleep schedule for an adult 450 may comprise sleep scheduling advice 424.

The baby sleep output 422 may comprise the future predicted sleep schedule for a baby 430. The recommended sleep schedule for an adult 450 may comprise a "coordinated sleep schedule", the recommended sleep schedule is coordinated with the future predicted sleep schedule for a baby 430.

The recommended sleep schedule for the adult 450 may comprise sleep sessions and awake sessions. Sleep sessions are periods of time the adult should be asleep (or in some examples, in bed trying to get to sleep) according to the coordinated sleep schedule. Sleep sessions may comprise periods of night-time sleep and/or periods of day-time sleep. Awake sessions are periods of time the adult should be awake (e.g. avoiding sleep) according to the coordinated sleep schedule.

The awake sessions may be configured to not overlap with the predicted sleep schedule for the baby. The awake sessions refer to awake sessions for the adult. In this way, the recommendation of the sleeping pattern for the adult may be configured such that the adult is to be awake when the baby is awake, and to be asleep when the baby is asleep. Where this is not possible, the coordinated sleep schedule may be configured to provide a compromise, such as maximising the amount of sleep scheduled for the adult which overlaps with the baby's sleep schedule.

The sleep analysis or sleep timing algorithm may generate an optimal control model in order to generate outputs. The optimal control model may use input metrics. Metrics act like variables in the model. The metrics are any suitable sleep metrics that can be recommended to the adult. Metrics may be one or more of: scheduled time for overnight sleep including sleep time and wake up time, scheduled time(s) for one or more naps, scheduled time(s) for unwinding before bed, scheduled time(s) for periods of activity, scheduled time(s) for caffeine intake, scheduled time(s) for breast pumping. The optimal control model may use key performance indicators. The key performance indicators may be one or more of: time spent asleep, number of times the adult was woken up, time spent awake during the night, quality of sleep. The time spent asleep is maximised. The number of times the adult is woken up is minimised. The time spent awake during the night is minimised. The quality of sleep is maximised.

In other examples, sleep analysis or sleep timing algorithms generate outputs using Al, machine learning or a neural network.

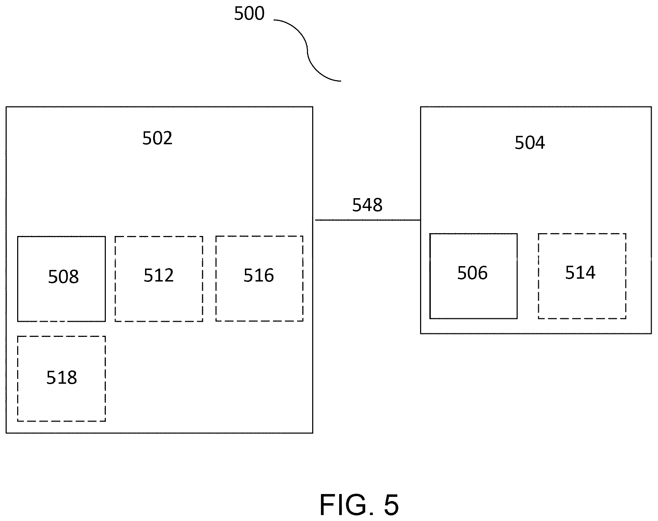

In the embodiment where the sleep tracking system comprises a breast pump, additional steps may be executed by the adult sleep analysis algorithm 416 or combined sleep analysis algorithm 418. The structure of this embodiment is described in relation to Figure 5. In this embodiment, the adult input data 402 further comprises breast pumping information. Breast pumping information may include one or more of: historical time periods of pumping, intensity setting information. Processing the adult input data and baby input data further comprises associating breast pumping information with the key performance indicators. As explained above, pumping too close to bedtime may impact the adult's sleep negatively. The adult sleep output further comprises scheduled time(s) for breast pumping 434.

The data processing steps involved depend on the embodiment of the invention. In a first embodiment where the sleep tracking system comprises the baby monitor or baby sleep tracking device and the application, the baby sleep analysis algorithm 414 receives baby input data 408 and generates baby sleep output 422.

In a second embodiment where the sleep tracking system comprises the adult sleep tracker device and the control device configured to run the application, the adult sleep analysis algorithm 416 receives the adult input data 402 and generates adult sleep output 420.

In a third embodiment where the sleep tracking system comprises the adult sleep tracker device, baby monitor or baby sleep tracking device and the control device configured to run the application, the baby sleep analysis algorithm 414 receives baby input data 408 and generates baby sleep output 422 and the adult sleep analysis algorithm 416 receives the adult input data 402 and generates adult sleep output 420. Additionally or alternatively in this embodiment, the combined sleep analysis algorithm 418 receives baby input data 408 and the adult input data 402 and generates adult sleep output 420 and baby sleep output 422.

### App

The application 110 may enable the adult to input any of the following: demographic information, wellbeing and health habits, hormonal cycle, personalised routines, sleep goals, sleep and activity schedules. This data is application adult data 406 which is adult input data 402. That is, adult application adult data 406 comprises one or more of: demographic information, wellbeing and health habits, hormonal cycle, personalised routines, sleep goals, sleep and activity schedules.

As described above, the application 110 may run on a control device 107. The control device 107 may comprise a component for the input of information. For example: a keyboard, keypad, drop down menu, button/s, voice command.

Demographic information can include information such as: age, gender, height, weight and ethnicity. Wellbeing and health habits can include information such as: diet information, caffeine intake, alcohol intake and smoking habits. Hormonal cycle can include information such as: date of last period, light to heavy flow and regularity of period. Personalised routine information can include information such as: times for planned cooking activities and times for planned social activities. Sleep goals can include information such as: ideal time spent asleep each night, ideal quality of sleep each night, ideal number of times woken up per night, ideal amount of time to go to sleep each night and ideal time to wake up each day. Sleep and activity schedules can include information such as: wake up time, time to go to bed, times for planned exercise.

As described above, the sleep tracking system 100 may include the adult sleep tracking device 102 and the control device 107 configured to run an app 110, such as a smartphone app, that is connected to the adult sleep tracking device, and the app displays the sleep scheduling advice 424.

The sleep scheduling advice 424 may include specific times during the day or night during which sleep is or is not recommended, based on information known about the adult, for example circadian rhythm. That is, the adult sleep analysis algorithm 416 or the combined sleep analysis algorithm 418 may determine the adult's circadian rhythm based on adult sleep tracker device data 404. The circadian rhythm of an adult is the sleep and awake cycle. Circadian rhythm is most easily changed one hour per night at a time. Therefore, if the adult sleep analysis algorithm 416 recommends a shift in the adult's sleep pattern, it will only shift the adult's sleep pattern by one hour per night for maximum effectiveness. In other words, the sleep scheduling advice 424 of consecutive days may be limited such that they differ by a maximum of 1 hour.

The sleep scheduling advice 424 includes specific times during the day or night during which activity is or is not recommended.

The sleep scheduling advice 424 may include specific times during the day or night during which drugs such as caffeine, alcohol or nicotine is or is not recommended. The sleep scheduling advice 424 may include advice on how long to sleep for a time period such as a 24h time period. Sleep scheduling will be described in more detail below.

As described above, the sleep tracking system 100 may include the baby monitor or sleep tracking device 104 and the control device 107 configured to run the app 110, such as a smartphone app, that is connected to the baby monitor or sleep tracking device 104. The application 110 may display the advice in a daily diary format showing times that sleep, such as naps and night-time sleep are recommended for the adult.

The application 110 may also display the predicted sleep schedule of a baby 430. The application 110, for the current day, may show when the baby awoke and when his next nap is scheduled. The application 110, for the current day, may show when the baby awoke and when his next nap is scheduled. Predictive advice will be explained in more detail below. In other words, the baby sleep output 422 may comprise the predicted sleep schedule of a baby 430.

The application 110 may allow for manual inputs of baby sleep data, for example when a baby has slept in a car seat away from their baby monitor or sleep tracking device. Application baby data 410 may comprise manual inputs of baby sleep data. Manual inputs of baby sleep data may be similar data to the baby monitor or baby sleep tracking device data 412.

The application 110, for the current day, may show various sleep insights 426, for example: when the adult woke in the morning, how long they slept for, how long it took for them to get to sleep, and when the adult should aim to sleep next. Adult sleep output 420 may comprise sleep insights 426.

The application 110 may display the predicted sleep schedule of a baby 430 in a daily diary format showing times that sleep is predicted, alongside the advice to the adult on when sleep for that adult is recommended. The application 110 may display the predicted sleep duration for the adult, based on expected sleep duration for the baby. The combined sleep analysis algorithm 418 may output predicted sleep schedule of a baby 430 based on baby input data 408, this output may be used to output sleep scheduling advice 424 and predicted sleep duration for the adult 428.

The application 110 may be provided with the birthdate or age of the baby and automatically generates sleep scheduling advice based on the standard developmental phases of the baby. The application 110 may be provided with the birthdate or age of the baby and may automatically generate general best-practise sleep advice based on the standard developmental phases of the baby. Application baby data 410 may comprise the birthdate or age of the baby. Different sleep patterns are associated with different standard developmental phases of the baby. For example, at 0-3 months babies typically sleep 14 to 17 hours every 24 hours whereas at 3-6 months babies typically sleep 12 to 15 hours every 24 hours.

Of course, any information described herein as being displayed on the app 110 may alternatively be displayed by any other suitable means. For example, the information may be displayed on the adult sleep tracker device 102 via a screen.

In some examples, the app allows the creation of a profile for a second adult, linked to the same account, that takes into account the second adult's situation and provides sleep scheduling advice for the whole family. For example if there's one stay at home parent and one parent working night shifts, the app can propose sleep schedules for both parents based on their baby and their own needs.

The application may allow multiple adults to see their baby's sleep information, whilst having tailored sleep advice and programmes for two adults (e.g. parents). In some examples, each adult has an adult tracking device. In other examples, one adult tracking device is shared between two adults.

Where there are two adults sleeping in one bed, the contactless sleep tracking sub-system can distinguish between the breathing sounds of the two adults. For example, by frequency filtering and separation. This separation between the two adults allows the sleep patterns of the two adults to be measured separately. In some examples, input data is saved and analysed for both adults and in other examples only the data of one adult is saved and analysed whilst the data for the other adult is discarded.

In a calibration mode, the sleep tracking system calibrates their normal, awake breathing sounds and normal breathing frequencies. The calibration of breathing sounds can be useful when there are two adults in one bed, the sleep tracking sub-system can filter out one person using frequency filtering. In other examples, the sleep tracking sub-system can record both adults and tracks both adults' sleeping patterns.

### Advice / algorithm

The advice is based on a sleep analysis or sleep timing algorithm that aims to increase the quantity of sleep enjoyed by that adult. Advice may be in the form of recommendations provided to the user. Advice may be given to the adult via the app, as explained herein. The terms "advice" and "recommendation/s" may be used interchangeably herein.

The sleep analysis or sleep timing algorithm aims to increase the quantity of sleep enjoyed by that adult, and decrease the likelihood of sleep inertia by waking them from sleep when the device predicts that they are in light-sleeping phase of their natural sleep cycle. The sleep analysis or sleep timing algorithm may achieve this via any known method, for example according to optimal control theory.

The sleep analysis or sleep timing algorithm may fit typical adult sleep cycle data with the adult input data 402. The data may be fitted by any known method, for example, linear regression. This advantageously allows the adult's sleep cycle to be predicted.

Typical adult sleep cycle data is based on known, typical sleep cycles of adults. Sleep cycles can be measured in time against the stage of sleep. The stages of sleep can be defined by two stages: rapid eye movement (REM) and non-rapid eye movement (NREM). NREM sleep may be divided into NREM stage 1, NREM stage 2 and NREM stage 3. Typical sleep cycle data may be obtained via sleep studies.

For a better fit to the user's own sleep data, the typical sleep cycle data used in the sleep analysis or sleep timing algorithm may be chosen based on the adult's demographic. For example, if the user is a female of 35 years old then typical sleep cycle data of other women in the 33-37 age range may be used. Application adult data 406 may comprise demographic data.

"Sleep scheduling advice 424" and "sleep window advice" may be referred to interchangeably herein. Sleep window advice may involve the recommendation to take one or more scheduled naps. In other words, sleep scheduling advice 424 may comprise scheduled naps. The recommendation to take one or more scheduled naps includes a time of day to take the nap(s) and the length of time of which to take the nap. If the sleep analysis or sleep timing algorithm detects the user is getting sleep per night below a pre-defined threshold, then the sleep analysis or sleep timing algorithm outputs advice for one or more naps in the day. The pre-defined threshold may be the recommended amount of sleep for that user based on their demographic. For example, 5 hours of sleep every 24 hours or 7 hours of sleep every 24 hours.

The naps may be recommended by the sleep analysis or sleep timing algorithm based on the user's circadian rhythm. For example, the algorithm may recommend naps after the user's cortisol levels have reduced. Cortisol levels reduce after the adult has been awake for a set amount of time, for most people this is in the afternoon. Therefore, in these cases naps would be recommended or scheduled for the afternoon. Some adults may have unusual sleeping patterns, in which case a different, suitable nap time is recommended.

Sleep window advice is adjusted based on feedback or ratings provided by the user, so it learns what advice is helpful/successful for that user over time, for example 'how do you feel after your nap?'. Application adult data 406 may comprise feedback from the adult on the most recent period(s) of sleep. If the feedback is negative after a nap, i.e. the user is not feeling particularly refreshed after their nap, then the sleep analysis or sleep timing algorithm may recommend a different time of day for the next nap (e.g. 3pm instead of 5pm) or may recommend a nap with a different length of time (e.g. 20 minutes instead of 1 hour). If the feedback is positive, i.e. the user feels refreshed after their nap, then the sleep analysis or sleep timing algorithm may continually recommend the same time of the day for the next nap and the same length of time to take the nap.

The sleep analysis or sleep timing algorithm may output predictive advice 432 to the user. Predictive advice 432 may comprise: sleep scheduling advice 424 and/or other predictive advice. Predictive advice 432 may relate to night-time sleep and/or predictive advice may relate to day-time nap or naps. That is, advice to the user by the sleep analysis or sleep timing algorithm on when to sleep (i.e. sleep scheduling) may involve any sleep periods within the day. Sleep periods typically include longer periods of night time sleep (for example, 3 to 8 hours) and shorter periods of day-time sleep (for example, 15 to 60 minutes).

The predictive advice 432 includes when to schedule periods of activity, based on understanding of how much sleep the individual has had. Based on the amount of the sleep the individual (the adult) has and optionally, also on the individual's circadian rhythm, the sleep analysis or sleep timing algorithm outputs predictive advice based on recommended activities. Predictive advice 432 comprises scheduled periods of activity for the adult.

When the adult is detected to have slept below the predefined threshold of recommended sleep, the sleep analysis or sleep timing algorithm may recommend low intensity activities. If the adult is able to breastfeed , the sleep analysis or sleep timing algorithm may recommend a breast pumping schedule based on the amount of sleep received by that adult. For example, if the adult has received below the predefined threshold amount of sleep when the sleep analysis or sleep timing algorithm may recommend a shorter amount of pumping as compared to a day when the adult had above the predefined threshold of sleep. This allows for increased pumping efficiency.

Predictive advice 432 takes into account the adult's hormonal changes, as determined automatically by a hormone tracking or analysis system or data input by the adult. The hormone tracking system may be based on input data from an adult's menstrual cycle, where that adult's hormones may be estimated over the course of a menstrual cycle. This allows for more accurate recommendations to be provided to the user.

Predictive advice 432 takes into account diary or activity inputs from, or associated with, the adult.

Adult input data 402 may comprise activity inputs. For example, if the adult has already done high intensity exercise or has already done a long breast pumping session, then the sleep analysis or sleep timing algorithm may not recommend these activities again in the following 24 hours. For high intensity activities (e.g. high intensity exercise or has already done a long breast pumping session) the sleep analysis or sleep timing algorithm may match these with the adult's recent sleep habits. When those sleep habits are repeated, the algorithm may recommend the high intensity activities again.

Activity inputs may be derived from a breast pump used by the adult. The activity input may comprise breast pumping information. The breast pumping information may include one or more of: historical time periods of pumping, intensity setting information and which breast left or right was used to pump. Intensity setting information a breast pump used by the adult may be labelled as high, medium or low intensity based on the level of vacuum applied to the breast over the pumping session.

Activity inputs may be derived from a wearable device, such as an activity tracker or smart watch, worn by the adult. The activity input may include one or more of: exercise duration, exercise intensity (e.g. measured by increase of user heart rate above resting heart rate), type of exercise (e.g. walking, running, swimming).

Predictive advice 432 may take into account when a baby wakes or cries, as determined by the baby monitoring device. The sleep analysis or sleep timing algorithm may, over time, form a model based on typical times when a baby cries or wakes. The combined sleep analysis algorithm 418 processes when the baby wakes or cries to generate the predicted sleep schedule of a baby 430. For example, the baby may often fall asleep within 20 minutes after a feeding session. For example, the baby may often start crying after 3 hours without a feeding session. For example, the baby may often wake up 2 hours after being laid down to sleep. This data is used to predict when the baby might cry or wake up. The times predicted when the baby may wake up or cry may be used in the sleep analysis or sleep timing algorithm to recommend predictive advice to the user. For instance, the data may be used to recommend sleep window advice to the user.

Predictive advice 432 may take into account how long it takes for a baby monitoring device (or sleep tracking device) to automatically and autonomously settle a crying baby back to sleep. The sleep analysis or sleep timing algorithm can therefore account for typical sleep that can be induced.

The combined sleep analysis algorithm 418 processes the length of time it takes to automatically and autonomously settle a crying baby back to sleep to generate the adult sleep output 420.

An algorithm (which can either form part of the sleep analysis or sleep timing algorithm or be a separate algorithm) is provided which supports getting women to sleep faster, by learning their unique sleep patterns and adjusting programmes to reduce sleep onset time. When the sleep and other activities are scheduled, for example according to the predictive advice described herein, this schedule helps the adult get to sleep faster.

Of course, the sleep analysis or sleep timing algorithm may also support any other adult (e.g. males) to get longer sleep and/or to get to sleep more quickly.

An algorithm (which can either form part of the sleep analysis or sleep timing algorithm or be a separate algorithm) is provided which adjusts programmes or tips to women's sleep needs, e.g. suggesting longer sleep programmes if they're in a particular stage of their hormonal cycle which they find more difficult to fall asleep in, or tips such as suggesting to wear cooler clothes.

### Key Feature B: Mother and baby sleep tracking system

A sleep tracking system including (i) a baby monitor or sleep tracking device configured to detect or track the times a baby wakes from sleep and cries and (ii) a separate adult sleep tracking device configured to detect or track the times an adult falls asleep;
and the system combines data from each device to generate and provide advice to the adult on when to schedule their sleep, in order to increase the amount of sleep enjoyed by that adult.

Optional features:
- the system combines data from each device to generate and provide advice to the adult on when to schedule the baby's sleep, in order to increase the amount of sleep enjoyed by the baby.
- Adult sleep tracking device includes a visual indicator to indicate a scheduled window is changing such as from active time to power nap time
- When adult and baby devices are brought within a certain range of each other, they 'sync', so lights and audio coordinate to avoid disruptive clashes

### Description of Key Feature B - Figure 5

An example configuration of the present invention is depicted in Figure 5. Figure 5 depicts a sleep tracking system 500 including (i) a baby monitor or sleep tracking device 504 configured to detect or track the times a baby wakes from sleep and cries and (ii) a separate adult sleep tracking device 502 configured to detect or track the times an adult falls asleep.

The system 500 may combine data from each device (the baby monitor or sleep tracking device 504 and adult sleep tracking device 502) to generate and provide advice to the adult on when to schedule their sleep, in order to increase the amount of sleep enjoyed by that adult. That is, sleep tracking system 500 may be configured to run one or more of: the combined sleep analysis algorithm 418, the baby sleep analysis algorithm 414 and the adult sleep analysis algorithm 416. The sleep tracking system 500 may comprise a processor to run the combined sleep analysis algorithm 418, the baby sleep analysis algorithm 414 and/or the adult sleep analysis algorithm 416. The baby monitor or sleep tracking device 104 and/or adult sleep tracking device 502 may comprise a processor configured to run the combined sleep analysis algorithm 418, the baby sleep analysis algorithm 414 and/or the adult sleep analysis algorithm 416.

The system 500 may combine data from each device (the baby monitor or sleep tracking device 504 and adult sleep tracking device 502) to generate and provide advice to the adult on when to schedule the baby's sleep, in order to increase the amount of sleep enjoyed by the baby. That is, the system 500 may be configured to run the combined sleep analysis algorithm 418.

In this example configuration, the baby monitor or sleep tracking device 504 and adult sleep tracking device 502 operate in a similar manner to as previously described in relation to Figure 1. The system 500 may combine data (from each device) to generate and provide advice to the adult on when to schedule their sleep or their baby's sleep according to the sleep analysis or sleep timing algorithm.

In the example configuration of Figure 5, the sleep tracking system 500 does not comprise an app. Advice to the adult relating to adult and/or baby sleep recommendations may be delivered by any other suitable means. In one example, advice is output via a display screen 516 which is part of the adult sleep tracker device. In this example, the adult sleep tracker device 502 comprises a display screen 516. In another example, advice is output via audible instructions delivered to the user via a speaker 518, the speaker forming part of the adult sleep tracker device 502. In this example, the adult sleep tracker device 502 comprises a speaker 518.

The adult sleep tracking device 502 may include a visual indicator to indicate a scheduled window is changing such as from active time to power nap time. A scheduled window refers to advice / recommendations generated by the sleep analysis or sleep timing algorithm as described above. That is, a scheduled window may refer to adult sleep output 420. In one example, the light source 512 may be configured to be the visual indicator of the adult sleep output 420. In this example, the visual indicator may comprise the colour of the light source 512. A first colour may indicate active time and a second colour indicates power nap time. Any nap may be referred to as a power nap. In another example, the display screen 516 provides the visual indicator. The screen 516 may display indications of the scheduled window changing such as from active time to power nap time.

In some examples, when adult and baby devices are brought within a certain range of each other, they 'sync', so lights and audio coordinate to avoid disruptive clashes. The adult sleep tracker device may be referred to as the "adult device" and the baby sleep tracking advice may be referred to as the "baby device". As described in relation to Figure 4, the sleep analysis or sleep timing algorithm may take input data 408, 402 from the adult sleep tracker device and the baby sleep tracking device respectively. In some examples, the adult sleep tracker device and the baby sleep tracking device may need to be brought within a certain range of each other to "sync" and for the sleep analysis or sleep timing algorithm to have all input data.

This example configuration is advantageous because it does not rely on an app. Therefore this configuration allows adults to reduce screen time on their phone before bed, this improves the adult's quality of sleep and reduces the time it takes for the adult to fall asleep.

The sleep tracking system 500 may be a network. The sleep tracking system 500 may be described as a sleep tracking ecosystem. The network comprising: an adult sleep tracker device 502 and a baby monitor or baby sleep tracking device 504. The adult sleep tracker device 502 and baby sleep tracking device 504 may be connected to each other via a communication link 548. This communication link may operate in a similar way to the communication link 148 described in relation to Figure 1. That is, the communication links 548 may be configured to transfer information. The communication link 548 may be wired or wireless connection. For example, the communication link 548 may comprise WiFi, Bluetooth, or any other wireless connection. Alternatively, the communication link 548 may comprise a USB cable.

The devices (adult sleep tracker device 502 and baby sleep tracking device 504) may be connected (e.g., networked) to each other in a Local Area Network (LAN), an intranet, an extranet, via the Internet or via Zigbee. The devices may each operate in the capacity of a server or a client machine in a client-server network environment, or as a peer machine in a peer-to-peer (or distributed) network environment.

### Key Feature C: Mother and baby sleep tracking system with sleep scheduling app

A sleep tracking system including:
(i) a sleep tracking device for an adult;
(ii) a sleep monitor or tracking device for a baby; and
(ii) an app, such as a smartphone app, that displays sleep scheduling advice for the adult, showing times that sleep, such as naps and night-time sleep is recommended based on (a) the personalised sleeping patterns of the adult measured or inferred by the sleep tracking device for the adult and (b) the personalised sleeping patterns of the baby measured or inferred by the monitor or sleep tracking device for the baby.

Optional features:
- sleep scheduling advice is in a daily diary format showing times that sleep, such as naps and night-time sleep is recommended
- the application also displays the predicted sleep schedule of a baby
- the application displays the predicted sleep schedule of a baby in a daily diary format showing times that sleep is predicted, alongside the advice to the adult on when sleep for that adult is recommended
- details of the phase of menstrual cycle the adult is in, entered manually or retrieved from an integrated product
- details of the phase of pregnancy the adult is in, entered manually or retrieved from an integrated product
- the perimenopausal or menopausal symptoms experienced by the adult, entered manually or retrieved from an integrated product
- the caffeine intake of the adult, entered manually or retrieved from an integrated product, (g) the physical activity, including vigorous exercise, of the adult, entered manually or retrieved from an integrated product
- scheduled appointments, entered manually or retrieved from an integrated calendar
- the circadian rhythm and sleep chronotype of the individual, entered manually or retrieved from an integrated product.

### Description of Key Feature C

In another example configuration of the present invention, there is provided a sleep tracking system. This example configuration may be described with reference to Figure 1.

There is provided a sleep tracking system 100 including: (i) a sleep tracking device for an adult 102; (ii) a sleep monitor or tracking device for a baby 104; and (ii) an app 110, such as a smartphone app. The app displays sleep scheduling advice for the adult, showing times that sleep, such as naps and night-time sleep is recommended based on (a) the personalised sleeping patterns of the adult measured or inferred by the sleep tracking device for the adult and (b) the personalised sleeping patterns of the baby measured or inferred by the monitor or sleep tracking device for the baby.

The terms "sleep tracking device for an adult" and "adult sleep tracker device" are interchangeable herein. Likewise, the terms "a sleep monitor or tracking device for a baby" and "baby monitor or baby sleep tracking device" are interchangeable herein.

The app 110 displays sleep scheduling advice for the adult according to the methods described above in relation to Figure 1. That is, the sleep scheduling advice is generated by the sleep analysis or sleep timing algorithm. As explained above, naps and night-time sleep is recommended based on the sleeping patterns of the adult and the sleeping patterns of the baby. Adult sleep patterns are measured or inferred by the sleep tracking device 102 for the adult, for example via the sleep sensing sub system 108. Baby sleep patterns are measured or inferred by the baby monitor or baby sleep tracking device 104, for example via the baby sleep tracking sub-system 106.

Sleep scheduling advice may be in a daily diary format showing times that sleep, such as naps and night-time sleep is recommended. That is, naps and night-time sleep for the adult. Sleep scheduling advice may be outputted to the user via the application 100. The application 110 may display the predicted sleep schedule of a baby. The application 110 may display the predicted sleep schedule of a baby in a daily diary format showing times that sleep is predicted, alongside the advice to the adult on when sleep for that adult is recommended.

In this example embodiment, there may be one or more additional input factors which may affect the user's sleep into the sleep analysis or sleep timing algorithm for improved accuracy in sleep planning of the adult. The first additional input factor is details of the phase of menstrual cycle the adult is in, entered manually or retrieved from an integrated product. This additional input factor may improve sleep planning by tracking how an adult exhibits different sleep patterns during a certain time in their menstrual cycle. For example, an adult may typically sleep for longer or shorter periods.

The second additional input factor is details of the phase of pregnancy the adult is in, entered manually or retrieved from an integrated product. This additional input factor may improve sleep planning using standard data based on sleep studies of other women can guide how sleep is typically affected during each phase of pregnancy.

The third additional input factor is the perimenopausal or menopausal symptoms experienced by the adult, entered manually or retrieved from an integrated product. This additional input factor may improve sleep planning by associating certain symptoms with sleep patterns.

The fourth additional input factor is the caffeine intake of the adult, entered manually or retrieved from an integrated product. This additional input factor may improve sleep planning by comparing the adults sleep patterns with caffeine intake, the advice may then be individualised based on the user's caffeine habits and ability to sleep after an intake of caffeine. For example, some users may cope with a caffeine intake 3 hours before bed and while others need 6 hours between caffeine intake and starting sleep.

The fifth additional input factor is the physical activity, including vigorous exercise, of the adult, entered manually or retrieved from an integrated product. This additional input factor may improve sleep planning by comparing the adult's sleep patterns with physical activity, the advice may then be individualised based on the user's physical activity routine and ability to sleep after physical activity. For some users, physical activity too close to bedtime may negatively impact sleep. For other users, physical activity close to bedtime may help induce sleep.

The sixth additional input factor is scheduled appointments, entered manually or retrieved from an integrated calendar. This input factor may override the normally generated recommendation. For example, the recommendation may normally generate a recommended bedtime of 10pm. However, the user may schedule a social appointment until 11pm that night. The recommendation is amended accordingly to fit with the user's schedule.

The seventh additional input factor is the circadian rhythm and sleep chronotype of the individual, entered manually or retrieved from an integrated product. If this information is available it may improve the accuracy of the algorithm. If this information is not available, it will be obtained over time via the tracking system.

The additional input factor data may be collected via an integrated product. Integrated product means that the product shares information with the sleep tracking system 100 such that the sleep analysis or sleep timing algorithm can receive this additional input factor data and then process it to output advice to the user. The integrated product may be any suitable product for measuring the additional input factor.

### Key Feature D: Sleep scheduling app

An app, such as a smartphone app, that displays sleep scheduling advice for an adult showing times that sleep, such as naps and night-time sleep, is recommended based on the personalised sleeping patterns of the adult and the personalised sleeping patterns of a baby cared for by the adult.

Optional features:
- sleep scheduling advice is in a daily diary format showing times that sleep, such as naps and night-time sleep is recommended
- the application also displays the predicted sleep schedule of a baby
- the application displays the predicted sleep schedule of a baby in a daily diary format showing times that sleep is predicted, alongside the advice to the adult on when sleep for that adult is recommended
- personalised sleeping patterns of the adult are discovered using a sleep tracking device for the adult
- personalised sleeping patterns of the baby are discovered using a sleep tracking device for the baby

### Description of Key Feature D

In another example configuration of the present invention, there is provided an app, such as a smartphone app, that displays sleep scheduling advice for an adult showing times that sleep, such as naps and night-time sleep, is recommended based on the personalised sleeping patterns of the adult and the personalised sleeping patterns of a baby cared for by the adult.

The app operates in a similar manner as described above. That is, the sleep scheduling advice may be in a daily diary format showing times that sleep, such as naps and night-time sleep is recommended. The application may also displays the predicted sleep schedule of a baby. The application may display the predicted sleep schedule of a baby in a daily diary format showing times that sleep is predicted, alongside the advice to the adult on when sleep for that adult is recommended.

In this example, the app provides the processing for runnning the sleep analysis or sleep timing algorithm. The app may be run on the control device which may be a local device (e.g. a handheld device) or the app may be substantially run on a server or in the cloud. The app may receive input data from any suitable source. For example, the user may input sleep data manually. For example, the user may estimate their own sleep patterns and/or their baby's sleep patterns. Alternatively, of course, personalised sleeping patterns of the adult may be discovered using a sleep tracking device for the adult and personalised sleeping patterns of the baby may be discovered using a sleep tracking device for the baby.

### Key Feature E: Mother and baby integrated breast pump and sleep tracking system

A mother and baby integrated care system including:
(i) a sleep tracking device configured to track a 'other's sleep;
(ii) a breast pump for use by the mother to express milk, the breast pump recording usage data;
(iii) a monitor or sleep tracking device for a baby; and
(iv) an app, such as a smartphone app, that displays sleep scheduling advice for the mother, together with breast pump usage data.

Optional features:
- breast pump usage data includes time and/or duration of milk pumping sessions
- breast pump usage data includes quantity of milk expressed
- breast pump usage data includes whether the left or right breast was used to express milk
- breast pump usage data includes predictive scheduling data indicating recommended times to express milk
- the app displays sleep scheduling advice for the mother, taking into account the sleep schedule of the baby
- the app displays sleep scheduling advice for the mother, taking into account the recommended milk expression schedule
- the sleep scheduling advice is in a daily diary format showing times that sleep, such as naps as night-time sleep is recommended, together with the predicted sleep schedule of the baby

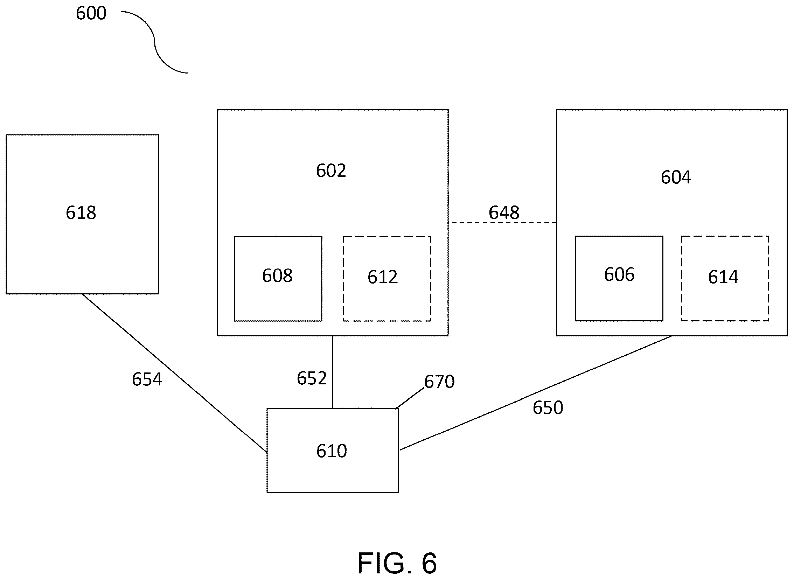

### Description of Key Feature E: Mother and baby integrated breast pump and sleep tracking system - Figure 6

Figure 6 depicts another example configuration of the present invention. In Figure 6, the sleep tracking system 600 comprises a breast pump.

There is provided a mother and baby integrated care system including: (i) a sleep tracking device configured to track a 'other's sleep; (ii) a breast pump 618 for use by the mother to express milk, the breast pump recording usage data; (iii) a monitor or sleep tracking device for a baby 604; and (iv) a control device 670 configured to run an app 610, such as a smartphone app, that displays sleep scheduling advice for the mother, together with breast pump usage data.

The mother and baby integrated care system may be referred to as the sleep tracking system 600. The sleep tracking device configured to track a 'other's sleep, may be referred to as an adult sleep tracker device 602. The monitor or sleep tracking device for a baby may be referred to as baby monitor or baby sleep tracking device 604.

The adult sleep tracker device 602, baby monitor or baby sleep tracking device 604 and the app 610 may operate in any manner in accordance with the above description.

The breast pump 618 is configured to generate pumping input data.

The breast pump 618 is configured to record breast pump usage data. Breast pump usage data may be input data into the sleep analysis or sleep timing algorithm. That is, adult input data 402 may comprise breast pump usage data and may be processed by the combined sleep analysis algorithm 418 and/or adult sleep analysis algorithm 416.

The breast pump usage data may include time and/or duration of milk pumping sessions. The breast pump usage data may include quantity of milk expressed. This may be measured by the breast pump 618 via any suitable means. For example: via an IR detector, a flow rate sensor or manual measurement by the user.

The breast pump usage data may include whether the left or right breast was used to express milk. This may be measured by the breast pump 618 via any suitable means. For example, recorded via manual input by the user via buttons on the breast pump 618.

In some examples, the app 610 may additionally display predictive scheduling data indicating recommended times to express milk. In these examples, the combined sleep analysis algorithm 418 and/or adult sleep analysis algorithm 416 processes the breast pump usage data with other adult input data 402 to output scheduled time(s) for breast pumping 434. That is, adult sleep output 420 may comprise scheduled time(s) for breast pumping 434. The scheduled time(s) for breast pumping 434 may be a recommended milk expression schedule (recommended times to express milk). In these examples, the scheduled time(s) for breast pumping 434 may take into other adult sleep output 420.

The app 610 may display sleep scheduling advice for the mother, tlking into account the sleep schedule of the baby. This may occur by any method previously described (e.g. via the sleep analysis or sleep timing algorithm). The app 610 may display sleep scheduling advice for the mother, taking into account the scheduled time(s) for breast pumping 434. That is, the sleep analysis or sleep timing algorithm may take the breast pump usage data (or a recommended milk expression schedule) as additional input data and adjust the outputted sleep advice accordingly.

For example, if a mother typically pumps most efficiently (volume of milk ÷ time spent pumping) in the morning then the sleep advice may be adjusted so that the mother wakes up before her optimal pumping time. In another example, if a pumping session too close to bedtime causes poor quality sleep then the recommended bedtime might be adjusted accordingly (or the recommended pumping time might be adjusted).

As described previously, the sleep scheduling advice may be in a daily diary format showing times that sleep, such as naps as night-time sleep is recommended, together with the predicted sleep schedule of the baby.

The sleep tracking system 600 is configured to generate a coordinated sleep schedule. Generating a coordinated sleep schedule may comprise receiving the generated pumping input data (from the breast pump 618). The generated pumping input data may comprise one or more of: pumping time, pumping duration, pumping intensity, and milk volume produced. Of course, other data related to breast pumps, pumping and milk production may also be used.

Generating a coordinated sleep schedule may comprise generating a breast pumping output. The breast pumping output comprises a recommended breast pumping schedule for the adult. A recommended breast pumping schedule for an adult may comprise a set of time periods recommended for the adult to use their breast pump.

The coordinated sleep schedule may comprise a recommended sleep schedule for the adult which comprises sleep sessions and awake sessions. The sleep sessions (of the adult) are configured to not overlap with the recommended breast pumping schedule.

The sleep tracking system 600 may generate a notification or alert in accordance with the coordinated sleep schedule. The notification or alert may be sent via the adult's app (e.g. via their smartphone) or via the adult sleep tracking device 602. The notification or alert may be a visual or audible alert. The notification or alert may comprise one or more messages: to start pumping, to stop pumping, a warning message that pumping should commence in a set amount of time.

The sleep tracking system 600 may be a network. The sleep tracking system 600 may be described as a sleep tracking ecosystem. The network comprising: an adult sleep tracker device 602, a baby monitor or baby sleep tracking device 604, a processor which runs an application 610 and a breast pump 618.

Each of the one or more devices may be connected to each other via one or more communication links 648, 650, 652 and 654. In particular, communication link 648 is optional.

The communication links 648, 650, 652 and 654 may be configured to transfer information. The communication links 648, 650, 652 and 654 may be wired or wireless connections. For example, the communication links 648, 650, 652 and 654 may comprise a USB cable. Alternatively the communication links 648, 650, 652 and 654 may comprise WiFi, bluetooth, or any other wireless connection.

The devices may be connected (e.g., networked) to each other in a Local Area Network (LAN), an intranet, an extranet, via the Internet or via Zigbee. The devices may each operate in the capacity of a server or a client machine in a client-server network environment, or as a peer machine in a peer-to-peer (or distributed) network environment.

### Key Feature F: Mother and baby sleep tracking system that integrates with a wearable device

A mother and baby integrated care system including:
(i) a sleep tracking device configured to track a mother's sleep;
(ii) a wearable activity monitor for tracking the physical activity of the mother;
(iii) a monitor or sleep tracking device for a baby; and
(iv) an app, such as a smartphone app, that displays sleep scheduling advice for the mother, where that advice takes into account physical activity of the mother.

Optional features:
- activity of the mother is derived from previous physical activity tracked by the activity monitor
- activity of the mother is derived from a prediction or schedule of when the mother is physically active and not sleeping or resting

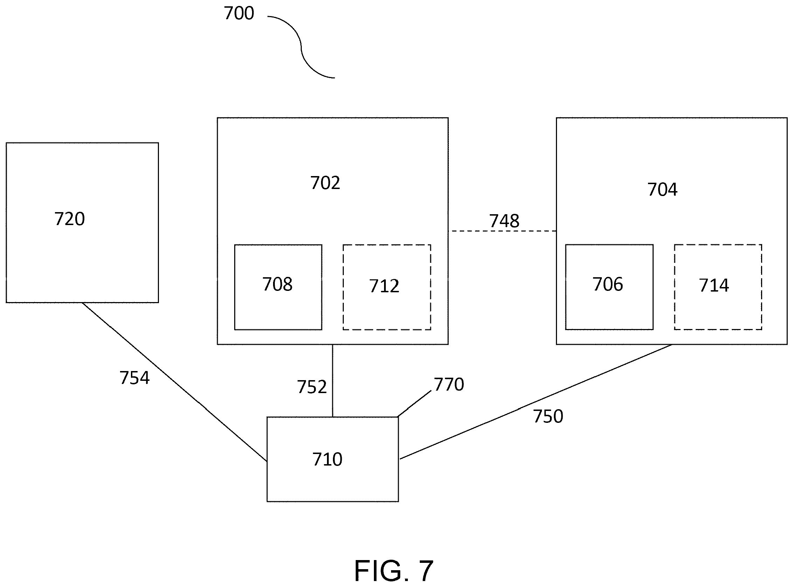

### Description of Key Feature F: Mother and baby sleep tracking system that integrates with a wearable device - Figure 7

Figure 7 depicts another example configuration of the present invention.

There is provided a mother and baby integrated care system including: a sleep tracking device configured to track a mother's sleep; a wearable activity monitor 720 for tracking the physical activity of the mother, a monitor or sleep tracking device for a baby; and a control device 770 configured to run an app 710, such as a smartphone app, that displays sleep scheduling advice for the mother, where that advice takes into account physical activity of the mother.

The mother and baby integrated care system may be referred to as the sleep tracking system 700. The sleep tracking device configured to track a mother's sleep, may be referred to as an adult sleep tracker device 702. The monitor or sleep tracking device for a baby may be referred to as baby monitor or baby sleep tracking device 704.

The activity of the mother may be derived from previous physical activity tracked by the activity monitor. That is, the activity monitor 720 may be configured to detect physical activity of the adult. The activity monitor 720 may be any device suitable for tracking physical activity of an adult. For example, a smart watch or a smartphone. The activity monitor 720 may be configured to detect heart rate of the adult.

In alternative examples, absent a wearable activity monitor 720 or if the wearable activity monitor 720 does not record data sufficiently, the activity of the mother may be derived from a prediction or schedule of when the mother is physically active and not sleeping or resting. That is, a baseline of data be recorded based on the adult's schedule.

The sleep scheduling advice includes specific times during the day or night during which activity is or is not recommended. This may be based on previous physical activity and how it affected the mother's sleep in previous nights.

In some examples, the wearable activity monitor 720 is configured to provide guided haptic feedback. The guided haptic feedback may be configured to relax the adult before sleep. The guided haptic feedback may guide an adult through a breathing pattern to allow for relaxation. This may be an alternative to light guided or audio guided relaxation. This is advantageous as it allows for an adult's eyes to be closed during the guided session.

Although described with reference to a mother, any other adult may also use the sleep tracking system 700 in the same way.

The sleep tracking system 700 may be a network. The sleep tracking system 700 may be described as a sleep tracking ecosystem. The network comprising: an adult sleep tracker device 702, a baby monitor or baby sleep tracking device 704 and/or a processor which runs an application 710. Each of the one or more devices may be connected to each other via one or more communication links 748, 750, 752 and 754. In particular, communication link 748 is optional.

The communication links 748, 750, 752, 754 may be configured to transfer information. The communication links 748, 750, 752, 754 may be wired or wireless connections. For example, the communication links 748, 750, 752, 754 may comprise a USB cable. Alternatively the communication links 748, 750, 752, 754 may comprise WiFi, bluetooth, or any other wireless connection.

The devices may be connected (e.g., networked) to each other in a Local Area Network (LAN), an intranet, an extranet, via the Internet or via Zigbee. The devices may each operate in the capacity of a server or a client machine in a client-server network environment, or as a peer machine in a peer-to-peer (or distributed) network environment.

### Key Feature G: Sleep tracking smart light and/or audio that reacts to sleep onset

A sleep tracking device configured to detect or track the times a person falls asleep and to provide a light and/or audio source that oscillates or varies in intensity with a period that, if matched by a person's breathing rate, tends to relax that person, and where the device dims or turns the light and/or audio source gradually off when it detects that the person has fallen asleep, or exhibits signs, such as altered breathing rate, that indicates that the person will shortly fall asleep.

### Description of Key Feature G: Sleep tracking smart light and/or audio that reacts to sleep onset

In another example configuration of the present invention, there is provided a sleep tracking device configured to detect or track the times a person falls asleep and to provide a light and/or audio source that oscillates or varies in intensity with a period that, if matched by a person's breathing rate, tends to relax that person, and where the device dims or turns the light and/or audio source gradually off when it detects that the person has fallen asleep, or exhibits signs, such as altered breathing rate, that indicates that the person will shortly fall asleep.

In this example configuration, the sleep tracking device is a standalone device comprising a light and/or audio source. As described above (in relation to Figure 1), a light source and/or audible source may be used which oscillates or varies in intensity with a period.

### Key Feature H: Sleep tracking smart light and/or audio that anticipates sleep onset

A sleep tracking device configured to detect or track the times an adult falls asleep and to provide a light and/or audio source that oscillates or varies in intensity with a period that, if matched by an adult's breathing rate, tends to relax that adult, and where the device stores or accesses a personal record of the adult's sleep routine, including how long they take to fall asleep, and automatically dims or reduces the intensity of the light and/or audio source over a time period derived from this personal record.

### Description of Key Feature H: Sleep tracking smart light and/or audio that anticipates sleep onset

In another example configuration of the present invention, there is provided a sleep tracking device configured to detect or track the times an adult falls asleep and to provide a light and/or audio source that oscillates or varies in intensity with a period that, if matched by an adult's breathing rate, tends to relax that adult, and where the device stores or accesses a personal record of the adult's sleep routine, including how long they take to fall asleep, and automatically dims or reduces the intensity of the light and/or audio source over a time period derived from this personal record.

In this example, the sleep tracking device anticipates when the adult might fall asleep and gradually reduces the intensity of the light and/or audio.

### Key Feature I: Sleep tracking smart light and/or audio that suggests day time naps

A sleep tracking system configured to detect or track the times a baby falls asleep and to provide a light and/or audio source that oscillates or varies in intensity with a period that, if matched by an adult's breathing rate, tends to relax that adult, and where the system is configured to detect or track if a baby is asleep, or infer that the baby is asleep based on the baby's previous sleep patterns, and the system automatically alters the appearance of the light and/or audio source to indicate to the adult when it is a suitable time to for the adult to take a nap, and for how long.

Optional feature:
- suitable time for the adult to take a nap is determined based on knowledge of how long it takes the individual to fall asleep.

### Description of key Feature I: Sleep tracking smart light and/or audio that suggests day time naps

In another example configuration of the present invention, there is provided a sleep tracking system configured to detect or track the times a baby falls asleep and to provide a light and/or audio source that oscillates or varies in intensity with a period that, if matched by a' adult's breathing rate, tends to relax that adult, and where the system is configured to detect or track if a baby is asleep, or infer that the baby is asleep based on the baby's previous sleep patterns, and the system automatically alters the appearance of the light and/or audio source to indicate to the adult when it is a suitable time to for the adult to take a nap, and for how long.

The suitable time for the adult to take a nap may be determined based on knowledge of how long it takes the individual to fall asleep.

This example sleep tracking system may work in a similar manner as described above.

### Key Feature J: Sleep tracking smart light that begins wake-up light program based on detected sleep cycle

Benefit: reduced grogginess/sleep inertia because we wake them in the right stage of their sleep cycle rather than just at a set time

A sleep tracking device configured to predict or determine a wake up window based on the detected sleep stage of an adult and to generate a light and/or audio source program derived to slowly awaken that adult, and where the device stores or accesses the adult's sleep routine, including how long they take to wake up, and automatically increases the intensity of the light and/or audio source over the wake up window such that the adult is awake near the end of the wake up window.

### Description of Key Feature J: Sleep tracking smart light that begins wake-up light program based on detected sleep cycle

In another example configuration of the present invention, there is provided a sleep tracking device configured to predict or determine a wake up window based on the detected sleep stage of an adult and to generate a light and/or audio source program derived to slowly awaken that adult, and where the device stores or accesses the adult's sleep routine, including how long they take to wake up, and automatically increases the intensity of the light and/or audio source over the wake up window such that the adult is awake near the end of the wake up window.

This feature of the sleep tracking system (wake-up light program based on the detected sleep cycle) may be applied to any sleep tracking system as described herein. For example, the system may determine whether it would be disruptive to wake the adult based on the detected sleep cycle. When the system has determined that it would be disruptive, the threshold time after which the adult will be notified that the baby is crying or awake may be increased. In this way, the system may be more resilient to a crying or awoken baby to a greater degree in order to protect the sleep health of the adult. In other words, the system may wake or alert the adult earlier than it would otherwise do, if it determines that the adult's sleep will not be disrupted or that the adult is already awake.

Benefit: reduced grogginess/sleep inertia because we wake them in the right stage of their sleep cycle rather than just at a set time.

### Key Feature K: Twist light to control light intensity & spread

A sleep tracking device with an integral light in a body of the device, configured so that twisting or rotating one part of the body relative to a different part of the body controls the light intensity emitted by the device.

Optional feature:
- twisting or rotating one part of the body relative to a different part of the body controls the spread of the light so as to be focused on a small area or a wide area to illuminate an entire room.

### Description of Key Feature K: Twist light to control light intensity & spread

In another example configuration of the present invention, there is provided a sleep tracking device with an integral light in a body of the device, configured so that twisting or rotating one part of the body relative to a different part of the body controls the light intensity emitted by the device.

The integral light in a body of the device is may be described as a light source. As described above, the light source can be physically adjusted to increase or decrease the amount of light visible, to make the light localised to one user, or to fill the whole room. The light source localised on one user allows that user to relax, for example by allowing the user to use the light source to read a book.

In one example, twisting or rotating one part of the body relative to a different part of the body controls the spread of the light so as to be focused on a small area or a wide area to illuminate an entire room.

Note that any Key Feature can be combined with any other Key Feature; any optional feature can be combined with any Key Feature and any other optional feature or suggested feature.

### Figure 8

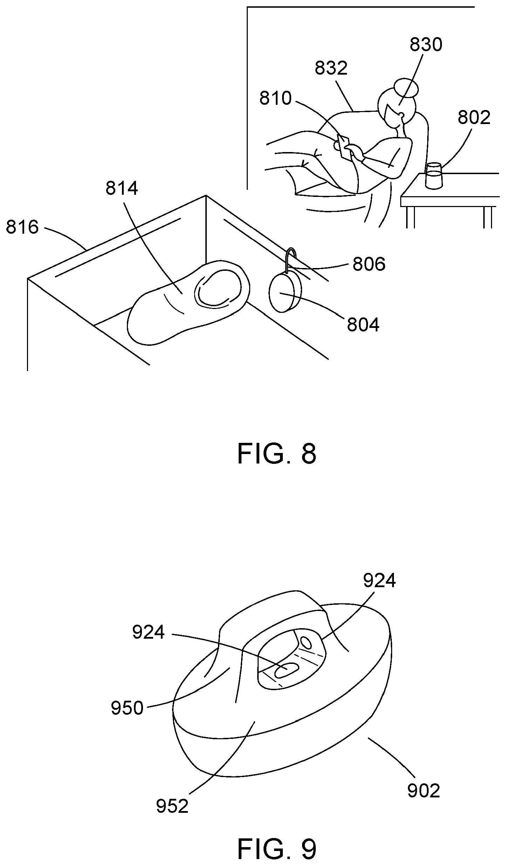

Figure 8 depicts an example environment for use of the sleep tracking system 100. Figure 8 depicts a baby 814 laying in a bed 816. In this example bed 816 is a crib. The baby monitor or baby sleep tracking device 804 may be positioned close to the baby 814. The baby monitor or baby sleep tracking device 804 may comprise an attachment portion 806. The baby monitor or baby sleep tracking device 804 may attached to a surface near the baby 814 via an attachment portion 806. The attachment portion 804 may comprise: a hook, one or more legs, a clamp and/or a deformable attachment. The baby monitor or baby sleep tracking device 804 is portable and may be battery operated.

Figure 8 depicts an adult 830 sitting on a sofa. The adult sleep tracker device 802 is positioned close to the adult 830. The adult sleep tracker device 802 may comprise an attachment portion. The attachment portion 806 is configured to attached the adult sleep tracker device 802 to a surface. The adult sleep tracker device 802 may rest on surface, as is depicted in Figure 8. The adult sleep tracker device 802 may alternatively be placed next to the adult's bed. The adult sleep tracker device 802 may be placed in any chosen location by the adult. The adult sleep tracker device 802 is portable and may be battery operated.

In alternative examples, the sleep tracking system comprises an electromechanically actuated bouncer or swing. The bouncer or swing may be suitable for a baby i.e. a baby bouncer or a baby swing. The bouncer or swing may be a freestanding unit or attach to a solid object such as a doorframe.

Initiating a settling routine may comprise controlling the electromechanically actuated bouncer or swing to oscillate. The oscillations or swings may help settle the baby back to sleep.

The baby sleep tracking device 804 may be releasably or permanently fixed to the electromechanically actuated bouncer or swing. The baby sleep tracking device may be physically integrated in the electromechanically actuated bouncer or swing. The baby sleep tracking device may be physically integrated by providing the baby sleep tracking device and the electromechanically actuated bouncer or swing, contained in the same housing. For example, the baby sleep tracking device may be contained in a housing of the electromechanically actuated bouncer or swing.

### Figure 9

Figure 9 depicts an adult sleep tracker device 902 according to an embodiment of the present invention. The adult sleep tracker device 902 configured to be wearable on an adult's finger. The adult sleep tracker device 902 may comprise a ring portion 950 and a body portion 952. The ring portion 950 is configured to be wearable on an adult's finger. The ring portion 952 is ring shaped or substantially circular to fit around an adult's finger. The body portion 952 houses the majority of the adult tracker device 902 electronics. The body portion 952 may be any suitable shape.

As described above, the adult sleep tracker device 902 may comprise a contact sleep tracking subsystem 924. In this embodiment, the adult sleep tracker device 902 comprises the contact sleep tracking sub-system 924 on the inner surface of the ring portion 950. That is, the adult sleep tracker device 902 may comprise one or more sensors on the inner surface of the ring portion 950. As described above, the contact sleep tracking sub-system 924 may comprise one or more of: a movement sensor, a pressure sensor, a temperature sensor. The inner surface of the ring portion comes into contact with the adult's finger and therefore the sensors may advantageously have close contact with the adult.

Additionally or alternatively, the adult sleep tracker device 902 comprises the contactless sleep tracking sub-system. The contactless sleep tracking sub-system may comprise one or more of: a radar system, a sonar system, a LIDAR system, a microphone, a camera, a heart rate sensor and a ballistocardiogram-based sensor. The adult sleep tracker device 902 may comprise the contactless sleep tracking sub-system in the body portion of the device.

In some examples, the adult sleep tracker device 902 is configured to provide guided haptic feedback. The haptic feedback may interface with the adult's finger and therefore be felt in the adult's hand. The contact sleep tracking sub-system 924 may be configured to provide the guided haptic feedback via one or more haptic actuators. The haptic actuators (not shown) may be located in the body portion 952 of the adult sleep tracker device 902. The guided haptic feedback may be configured to relax the adult before sleep. For example, the guided haptic feedback may guide an adult through a breathing pattern to allow for relaxation. By providing haptic feedback which is related to a breathing pattern, the guided haptic feedback may allow the user to focus on breathing and slow breathing to relax. This may be an alternative to light guided guided relaxation. This is advantageous as it allows for an adult's eyes to be closed during the guided session.

### Figure 10A and Figure 10B

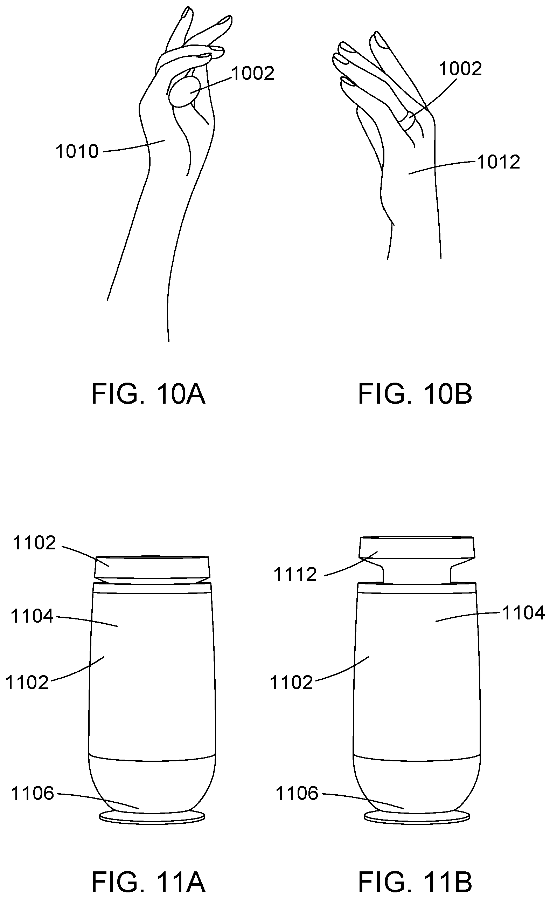

Figures 10A and 10B depict two views the adult sleep tracker device 1002. The adult sleep tracker device 1002 may be the adult sleep tracker device 902 configured to be wearable on an adult's finger. The adult sleep tracker device 1002 may comprise a ring portion and a body portion. Figure 10A depicts a first hand 1010 viewed with the palm of the hand visible. The adult sleep tracker device 1002 worn by on the finger of the hand. The adult sleep tracker device 1002 may be configured such that the body portion rests on the palm of the adult's hand.

Figure 10B depicts a second hand 1012 viewed with the back of the hand visible. The adult sleep tracker device 1002 may be configured such that the body portion rests on the palm of the adult's hand, therefore only the ring portion is visible.

### Figure 11A and Figure 11B

Figures 11A and 11B depict an adult sleep tracker device 1102 in alternative embodiment to Figures 9-10B. In this embodiment, the adult sleep tracker device 1102 is configured to rest on a surface. The adult sleep tracker device 1102 may comprise a body portion 1104 and a stand portion 1106. The body portion 1104 may the majority of the electronics of the adult sleep tracker device 1102. The stand portion 1106 is configured to allow the adult sleep tracker device 1102 to stand on a flat surface. The adult sleep tracker device 1102 may rest on any flat surface. For example, a table or a bed headboard. The adult sleep tracker device 1102 may comprise a light source 1112, in accordance with the description above.

Figure 11A depicts the adult sleep tracker device 1102 where the light source 1112 is in a first position. Figure 11B depicts the adult sleep tracker device 1102 where the light source 1112 is in a second position. The light source 1112 is movable from the first position to the second position. The light source 1112 may be movable by the user. For example, by pressing the light source 1112 or another button on the adult sleep tracker device. The light source may automatically move between the first position and the second position. In embodiments where the light source is configured to gradually increases its brightness when the adult is scheduled to wake up, the light source may gradually move from the first position to the second position. In this way, the light source gradually appears and therefore causes a change of depth in the light across the room, thus mimicking the rising of the Sun and causing the adult to feel refreshed.

Of course, the adult sleep tracker device 902, 1002, 1102 may be applied to the invention as described in relation to Figures 1 to 8.

### Figure 12

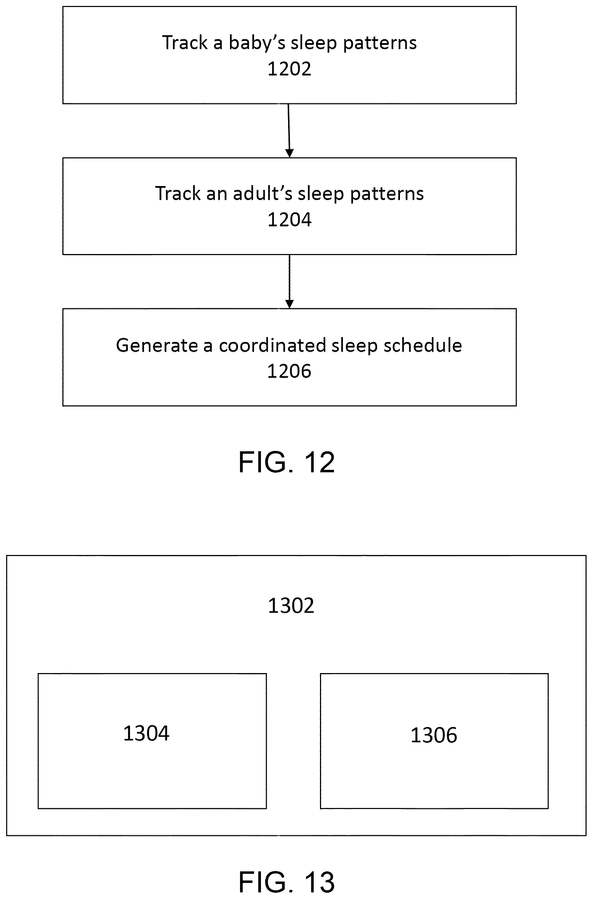

Figure 12 depicts a method which may be executed by one or more processors, the one or more processors part of the sleep tracking system according to the present invention.

In a first step a baby's sleep patterns 1202 are tracked. In a second step an adult's sleep patterns 1204 are tracked. The first step and the second step 1202, 1204 may occur at the same time or at different times. In a third step a coordinated sleep schedule 1206 is generated. The coordinated sleep schedule may be coordinated in the sense that the adult and baby sleep schedules (sleep sessions and awake sessions) are optimised taking into account both the baby's and the adult's sleep. For example, the coordinated sleep schedule may provide for maximum sleep quality of the adult, maximum sleep quality of the baby, and/or a maximum combined sleep quality of the baby and the adult.

The coordinated sleep schedule 1212 may be described as an adult sleep output and a baby sleep output, the baby sleep output comprising a future predicted sleep schedule for a baby, and the adult sleep output comprises a recommended sleep schedule for an adult. The recommended sleep schedule for the adult may comprise sleep sessions and awake sessions.

The sleep tracking system comprising a baby sleep tracking device and an adult sleep tracker device. The baby sleep tracking device is configured to track a baby's sleep patterns. The baby sleep tracking device may be the baby sleep tracking device of any of Figures 1 and/or 3 to 8.

The adult sleep tracker device is configured to track an adult's sleep patterns. The adult sleep tracker device may be the adult sleep tracking device of any of Figures 1, 2, 4 to 11B. The sleep tracking system is configured to generate a coordinated sleep schedule.

### Figure 13

Figure 13 depicts an exemplary computing device 1302. A computing device comprises a processor 1304 and a memory 1306. The memory 1306 may be a non-transient computer readable medium comprising instructions that when executed by the processor 1304 cause the processor to carry out one or more steps of the methods described herein and/or operated one or more of the systems described herein.

Features of the above aspects can be combined in any suitable manner. It will be understood that the above description is of specific embodiments by way of aspect only and that many modifications and alterations will be within the skilled person's reach.

## Claims

1. A sleep tracking system comprising: a baby sleep tracking device (604); an adult sleep tracker device (602);
wherein the baby sleep tracking device is configured to track a baby's sleep patterns, the adult sleep tracker device is configured to track an adult's sleep patterns, and the sleep tracking system is configured to generate a coordinated sleep schedule, wherein:
generating a coordinated sleep schedule comprises:
receiving adult input data;
receiving baby input data;
processing the adult input data and baby input data using key performance indicators; **characterized in that**:
a) the system further comprises a breast pump (618);b) generating a coordinated sleep schedule further comprises:
generating an adult sleep output and a baby sleep output, wherein the baby sleep output comprises a future predicted sleep schedule for a baby, and the adult sleep output comprises a recommended sleep schedule for an adult comprising sleep sessions and awake sessions;
and further **characterized in that**:
the breast pump is configured to generate pumping input data;
generating a coordinated sleep schedule comprises receiving the generated pumping input data;
the generated pumping input data comprises one or more of: pumping time, pumping duration, pumping intensity, and milk volume produced;
generating a coordinated sleep schedule comprises generating a breast pumping output; and
the breast pumping output comprises a recommended breast pumping schedule for an adult,
wherein the sleep sessions are configured to not overlap with the recommended breast pumping schedule.

2. The sleep tracking system of claim 1 further comprising a control device configured to run an application.

3. The sleep tracking system of any preceding claim, wherein the adult sleep tracker device and/or baby sleep tracking device comprise a contactless sleep tracking sub-system.

4. The sleep tracking system of claim 3, wherein the contactless sleep tracking sub-system comprises one or more of: a radar system, a sonar system, a LIDAR system, a microphone, a camera, a heart rate sensor and a ballistocardiogram-based sensor.

5. The sleep tracking system of any preceding claim, wherein the adult sleep tracker device and/or the baby sleep tracking device comprise a contact sleep tracking sub-system.

6. The sleep tracking system of claim 5, wherein the contact-based sleep tracking sub-system comprises one or more of: one or more movement sensors, one or more pressure sensors, one or more temperature sensors and one or more heart rate sensors.

7. The sleep tracking system of any preceding claim, wherein the adult sleep tracker device and/or the baby sleep tracking device further comprises one or more of: a light source, an audio source and one or more buttons.

8. The sleep tracking system of any preceding claim, wherein the baby sleep tracking device is configured to:
detect that a baby is awake or crying;
initiate a settling routine to settle the baby back to sleep;
detect that the baby is no longer awake or crying;
stop the settling routine.

9. The sleep tracking system of any preceding claim, wherein the baby sleep tracking device is configured to:
detect that a baby is awake or crying;
initiate a settling routine to settle the baby back to sleep;
detect that the baby is still awake or crying after a pre-defined time threshold;
trigger an alert at one or both of the adult sleep tracker device or the control device.

10. The sleep tracking system of claim 8 or claim 9, further comprising an electromechanically actuated bouncer or swing, and wherein initiating the settling routine comprises controlling the electromechanically actuated bouncer or swing to oscillate, and optionally wherein the baby sleep tracking device is physically integrated in the electromechanically actuated bouncer or swing.

11. The sleep tracking system of claim 10, wherein the baby sleep tracking device is releasably or permanently fixed to the electromechanically actuated bouncer or swing.

12. The sleep tracking system of any preceding claim, wherein the awake sessions are configured to not overlap with the predicted sleep schedule for the baby.

13. The sleep tracking system of any preceding claim further configured to generate a notification or alert in accordance with the coordinated sleep schedule.

14. The sleep tracking system of any preceding claim, wherein the adult sleep tracker device comprises a wearable activity monitor.

15. The sleep tracking system of claim 14, wherein the wearable activity monitor is a ring.

## Patentansprüche

1. Schlafverfolgungssystem, umfassend: ein Gerät (604) zur Verfolgung eines Babyschlafs; ein Erwachsenenschlaf-Trackergerät (602);
wobei das Gerät zur Verfolgung des Babyschlafs konfiguriert ist, um Schlafmuster eines Babys zu verfolgen, das Erwachsenenschlaf-Trackergerät konfiguriert ist, um die Schlafmuster eines Erwachsenen zu verfolgen, und das Schlafverfolgungssystem konfiguriert ist, um einen koordinierten Schlafplan zu erstellen, wobei:
ein Erstellen eines koordinierten Schlafplans umfasst:
Empfangen von Erwachsenen-Eingabedaten;
Empfangen von Baby-Eingabedaten;
Verarbeiten der Erwachsenen-Eingabedaten und der Baby-Eingabedaten unter Verwendung von Schlüsselleistungsindikatoren;
**dadurch gekennzeichnet, dass**:
a) das System ferner eine Milchpumpe (618) aufweist;
b) das Erstellen eines koordinierten Schlafplans ferner umfasst:
Erstellen einer Erwachsenen-Schlafausgabe und einer Baby-Schlafausgabe, wobei die Baby-Schlafausgabe einen zukünftigen vorhergesagten Baby-Schlafplan umfasst und die Erwachsenen-Schlafausgabe einen empfohlenen Erwachsenen-Schlafplan mit Schlafphasen und Wachphasen umfasst; und
ferner **dadurch gekennzeichnet, dass**:
die Milchpumpe konfiguriert ist, um Pumpeingabedaten zu erzeug;
das Erstellen eines koordinierten Schlafplans ein Empfangen der erzeugten Pumpeingabedaten umfasst;
die erzeugten Pumpeingangsdaten eines oder mehrere der folgenden Elemente umfassen: Pumpzeit, Pumpdauer, Pumpintensität und produziertes Milchvolumen;
das Erstellen eines koordinierten Schlafplans ein Erstellen einer Brustpumpausgabe umfasst; und
die Brustpumpausgabe einen empfohlenen Brustpumpplan für einen Erwachsenen umfasst,
wobei die Schlafphasen so konfiguriert sind, dass sie sich mit dem empfohlenen Brustpumpplan nicht überschneiden.

2. Schlafverfolgungssystem nach Anspruch 1, ferner umfassend eine Steuervorrichtung, die zum Ausführen einer Anwendung konfiguriert ist.

3. Schlafverfolgungssystem nach einem der vorstehenden Ansprüche, wobei das Erwachsenenschlaf-Trackergerät und/oder Gerät zur Verfolgung eines Babyschlafs ein kontaktloses Schlafverfolgung-Subsystem umfasst.

4. Schlafverfolgungssystem nach Anspruch 3, wobei das kontaktlose Schlafverfolgung-Subsystem eines oder mehrere der folgenden Elemente umfasst: ein Radarsystem, ein Sonarsystem, ein LIDAR-System, ein Mikrofon, eine Kamera, einen Herzfrequenzsensor und einen auf einem Ballistokardiogramm basierenden Sensor.

5. Schlafverfolgungssystem nach einem der vorstehenden Ansprüche, wobei das Erwachsenenschlaf-Trackergerät und/oder das Gerät zur Verfolgung eines Babyschlafs ein kontaktbasiertes Schlafverfolgung-Subsystem umfasst.

6. Schlafverfolgungssystem nach Anspruch 5, wobei das kontaktbasierte Schlafverfolgung-Subsystem eines oder mehrere der folgenden Elemente umfasst: einen oder mehrere Bewegungssensoren, einen oder mehrere Drucksensoren, einen oder mehrere Temperatursensoren und einen oder mehrere Herzfrequenzsensoren.

7. Schlafverfolgungssystem nach einem der vorstehenden Ansprüche, wobei das Erwachsenenschlaf-Trackergerät und/oder das das Gerät zur Verfolgung eines Babyschlafs ferner eines oder mehrere der folgenden Elemente umfasst: eine Lichtquelle, eine Audioquelle und eine oder mehrere Tasten.

8. Schlafverfolgungssystem nach einem der vorstehenden Ansprüche, wobei das das Gerät zur Verfolgung eines Babyschlafs konfiguriert ist, um:
zu detektieren, dass ein Baby wach ist oder weint;
eine Beruhigungsroutine einzuleiten, um das Baby wieder in den Schlaf zu versetzen;
zu detektieren, dass das Baby nicht mehr wach ist oder weint;
die Beruhigungsroutine zu beenden.

9. Schlafverfolgungssystem nach einem der vorstehenden Ansprüche, wobei das Gerät zur Verfolgung eines Babyschlafs konfiguriert ist, ums
zu detektieren, dass ein Baby wach ist oder weint;
eine Beruhigungsroutine einzuleiten, um das Baby wieder in den Schlaf zu versetzen;
zu detektieren, dass das Baby nach einer vordefinierten Zeitspanne immer noch wach ist oder weint;
einen Alarm an einem oder beiden der Erwachsenenschlaf-Trackergeräte oder der Steuervorrichtung auszulösen.

10. Schlafverfolgungssystem nach Anspruch 8 oder Anspruch 9, ferner umfassend eine elektromechanisch betätigte Wippe oder Schaukel, wobei ein Einleiten der Beruhigungsroutine ein Steuern der elektromechanisch betätigten Wippe oder Schaukel zum Schwingen umfasst und wobei das das Gerät zur Verfolgung eines Babyschlafs optional physisch in die elektromechanisch betätigte Wippe oder Schaukel integriert ist.

11. Schlafverfolgungssystem nach Anspruch 10, wobei das das Gerät zur Verfolgung eines Babyschlafs lösbar oder dauerhaft an der elektromechanisch betätigten Wippe oder Schaukel befestigt ist.

12. Schlafverfolgungssystem nach einem der vorstehenden Ansprüche, wobei die Wachphasen so konfiguriert sind, dass sie sich nicht mit dem vorhergesagten Schlafplan für das Baby überschneiden.

13. Schlafverfolgungssystem nach einem der vorstehenden Ansprüche, wobei es ferner konfiguriert ist, um eine Benachrichtigung oder einen Alarm gemäß dem koordinierten Schlafplan zu erzeugen.

14. Schlafverfolgungssystem nach einem der vorstehenden Ansprüche, wobei das Erwachsenenschlaf-Trackergerät einen tragbaren Aktivitätsmonitor aufweist.

15. Schlafverfolgungssystem nach Anspruch 14, wobei der tragbare Aktivitätsmonitor ein Ring ist.

## Revendications

1. Système de suivi du sommeil comprenant : un dispositif de suivi du sommeil d'un bébé (604) ; un dispositif de suivi du sommeil d'un adulte (602) ;
dans lequel le dispositif de suivi du sommeil du bébé est configuré pour suivre les habitudes de sommeil d'un bébé, le dispositif de suivi du sommeil de l'adulte est configuré pour suivre les habitudes de sommeil d'un adulte, et le système de suivi du sommeil est configuré pour générer un programme de sommeil coordonné,dans lequel :
la génération d'un programme de sommeil coordonné comprend :
la réception de données d'entrée relatives à l'adulte ;
la réception de données d'entrée relatives au bébé ;
le traitement des données d'entrée de l'adulte et du bébé à l'aide d'indicateurs de performance clés; **caractérisé en ce que** :
a) le système comprend en outre un tire-lait (618) ; b) la génération d'un programme de sommeil coordonné comprend en outre :
la génération d'une sortie de sommeil pour un adulte et d'une sortie de sommeil pour un bébé, dans laquelle la sortie de sommeil du bébé comprend un programme de sommeil prévisionnel pour un bébé, et la sortie de sommeil de l'adulte comprend un programme de sommeil recommandé pour un adulte, comprenant des périodes de sommeil et des périodes d'éveil ;
et **caractérisé en outre en ce que** :
le tire-lait est configuré pour générer des données d'entrée de pompage ;
la génération d'un programme de sommeil coordonné comprend la réception des données d'entrée de pompage générées ;
les données d'entrée de pompage comprennent une ou plusieurs des informations suivantes :
heure de pompage, durée de pompage, intensité de pompage et volume de lait produit ;
la génération d'un programme de sommeil coordonné comprend la génération d'une sortie de pompage mammaire; et la sortie de pompage mammaire comprend un programme de pompage mammaire recommandé pour un adulte,
dans lequel les périodes de sommeil sont configurées de manière à ne pas chevaucher le programme de pompage mammaire recommandé.

2. Le système de suivi du sommeil selon la revendication 1, comprenant en outre un dispositif de commande configuré pour exécuter une application.

3. Le système de suivi du sommeil selon l'une quelconque des revendications précédentes, dans lequel le dispositif de suivi du sommeil de l'adulte et/ou le dispositif de suivi du sommeil du bébé comprend un sous-système de suivi du sommeil sans contact.

4. Le système de suivi du sommeil selon la revendication 3, dans lequel le sous-système de suivi du sommeil sans contact comprend un ou plusieurs des éléments suivants :
un système radar, un système sonar, un système LIDAR, un microphone, une caméra, un capteur de fréquence cardiaque et un capteur basé sur la ballistocardiographie.

5. Le système de suivi du sommeil selon l'une quelconque des revendications précédentes, dans lequel le dispositif de suivi du sommeil de l'adulte et/ou du bébé comprend un sous-système de suivi du sommeil par contact.

6. Le système de suivi du sommeil selon la revendication 5, dans lequel le sous-système de suivi du sommeil par contact comprend un ou plusieurs des éléments suivants : un ou plusieurs capteurs de mouvement, un ou plusieurs capteurs de pression, un ou plusieurs capteurs de température et un ou plusieurs capteurs de fréquence cardiaque.

7. Le système de suivi du sommeil selon l'une quelconque des revendications précédentes, dans lequel le dispositif de suivi du sommeil de l'adulte et/ou du bébé comprend en outre un ou plusieurs des éléments suivants : une source lumineuse, une source sonore et un ou plusieurs boutons.

8. Le système de suivi du sommeil selon l'une quelconque des revendications précédentes, dans lequel le dispositif de suivi du sommeil du bébé est configuré pour :
détecter qu'un bébé est réveillé ou pleure ;
initier une routine d'apaisement pour aider le bébé à se rendormir ;
détecter que le bébé n'est plus éveillé ou ne pleure plus ;
arrêter la routine d'apaisement.

9. Le système de suivi du sommeil selon l'une quelconque des revendications précédentes, dans lequel le dispositif de suivi du sommeil du bébé est configuré pour :
détecter qu'un bébé est réveillé ou pleure ;
initier une routine d'apaisement pour aider le bébé à se rendormir ;
détecter que le bébé est encore éveillé ou pleure après un seuil de temps prédéfini ;
déclencher une alerte sur le dispositif de suivi du sommeil de l'adulte et/ou sur le dispositif de commande.

10. Le système de suivi du sommeil selon l'une quelconque des revendications 8 ou 9, comprenant en outre un transat ou une balançoire à commande électromécanique, et dans lequel l'initiation de la routine d'apaisement comprend le contrôle du mouvement oscillant d'un transat ou d'une balançoire, le dispositif de suivi du sommeil du bébé pouvant être intégré physiquement audit transat ou à ladite balançoire à commande électromécanique.

11. Le système de suivi du sommeil selon la revendication 10, dans lequel le dispositif de suivi du sommeil du bébé est fixé de manière amovible ou permanente au transat ou à la balançoire électromécanique.

12. Le système de suivi du sommeil selon l'une quelconque des revendications précédentes, dans lequel les périodes d'éveil sont configurées de manière à ne pas chevaucher le programme de sommeil prévisionnel du bébé.

13. Le système de suivi du sommeil selon l'une quelconque des revendications précédentes, en outre configuré pour générer une notification ou une alerte conformément au programme de sommeil coordonné.

14. Le système de suivi du sommeil selon l'une quelconque des revendications précédentes, dans lequel le dispositif de suivi du sommeil de l'adulte comprend un capteur d'activité portable.

15. Le système de suivi du sommeil selon la revendication 14, dans lequel le capteur d'activité portable est une bague.
